# EUROPEAN PATENT SPECIFICATION

(11) **EP 1 144 692 B1**
(45) Date of publication and mention of the grant of the patent: **11.02.2004**
(21) Application number: 99973074.0
(22) Date of filing: 30.11.1999
(51) Int. Cl.: C12Q 1/68, C12N 15/10

(54) **METHOD FOR SEPARATING NUCLEIC ACIDS INTO POPULATIONS**
METHODE ZUR TRENNUNG VON NUKLEINSÄUREN IN POPULATIONEN
PROCEDE DE SEPARATION D'ACIDES NUCLEIQUES DANS DES POPULATIONS

(30) Priority: 30.11.1998 GB 9826247
(43) Date of publication of application: 17.10.2001
(73) Proprietor: Axis-Shield PoC AS, 0566 Oslo (NO)
(72) Inventor: IHLE, Oystein, N-1314 Skui (NO); MICHAELSEN, Terje, Einar, N-1481 Hagan (NO); JOHNE, Berit, N-0389 Oslo (NO)
(74) Representative: Webber, Philip Michael
(86) International application number: PCT/GB1999/003995
(87) International publication number: WO 2000/032812

(56) References cited:
- EP-A- 0 872 559
- WO-A-85/04674
- WO-A-90/12115
- WO-A-97/28282
- FR-A- 2 678 639
- US-A- 5 602 300
- US-A- 5 741 678

## Description

The present invention relates to a method for at least partially separating nucleic acid fragments. In particular, it relates to a method for separating from a population of nucleic acid molecules those which are tagged with a protein which can be immobilised on a matrix which selectively binds proteins.

Manipulation and handling of DNA is central to most biotechnology techniques. The manipulation of DNA typically involves endonuclease digestion using specific restriction enzymes which cut the DNA into fragments, followed by purification of the DNA fragments, insertion of the required fragment into cloning vectors and transfer of these vectors into non-native hosts for transcription, and optionally translation, thereby providing valuable biological information, and/or expression of the inserted DNA into a product, such as a therapeutic product. This enables, for example, eucaryotic proteins to be expressed in bacteria. The ability to cut and join DNA molecules or fragments is central to modern biotechnology. Often, these DNA fragments are generated by the polymerase chain reaction (PCR), which is now a common tool both in research and industrial biotechnology. This method enables specific DNA molecules to be amplified by means of specific short nucleic acid primers to produce large quantities of DNA which can then be further manipulated for example using the aforementioned cloning techniques.

Methods of nucleic acid manipulation which involve the use of DNA molecules generated for example by means of the aforementioned restriction enzyme cutting or by PCR are often inefficient due at least in part to the presence of unwanted DNA molecules. Such 'unwanted' molecules include for example vector DNA resulting from excision, particularly incomplete excision, of an inserted DNA fragment from a recombinant molecule, partially digested restriction fragments or other by-products of restriction enzyme cutting of DNA molecules, excess PCR primers, incorrectly ligated nucleic acid molecules which are the by-products of nucleic acid manipulation, and PCR products which are the result of misannealing of a PCR primer with the template nucleic acid. The quality of primary end product DNA is crucial for the success of downstream manipulations such as ligation and transformation of bacterial or eukaryotic host cells. The ability to separate mixtures of nucleic acid molecules, such as mixtures of DNA molecules or DNA fragments into different populations and thereby remove what is considered to be the 'unwanted' or contaminating population from the desired or target nucleic acid molecule would thus enhance the efficiency of further processing or downstream steps using such generated nucleic acid molecules.

Methods for purifying nucleic acid molecules as a class are known in the art. There are however limited methods available which can separate mixtures of nucleic acid molecules such as mixtures comprising several different DNA molecules into different populations. Generally, these methods rely on separation of nucleic acid molecules, or fragments, according to size, for example by means of electrophoresis through agarose or polyacrylamide gels, followed by purification of the desired molecule or fragment. These methods have a number of drawbacks. One limiting factor is the capacity of the gel itself, which limits the amount of DNA which can be separated. The DNA needs to be visualised in the gel, generally by way of staining with ethidium bromide. Aside from being toxic to the operator, this can contribute to a reduction in the quality of the nucleic acid, so that performance in downstream applications can be poor. Recovery of nucleic acid molecules fractionated by gel electrophoresis is also inefficient leading to significant losses, often of at least 20% of DNA. Gel electrophoretic methods are also time consuming. DNA is a fragile molecule and is vulnerable to attack by exo and endonucleases. The comparatively long process of electrophoretic separation, during which the DNA is vulnerable to degradation, can thus be detrimental to the integrity of the DNA, and affect the efficiency of downstream processes. Such separation methods are thus inefficient and costly.

EP-A-0 872 559 discloses a method for the simultaneous detection of polygenes. In particular, Figure 1 discloses the use of biotin-labelled primers to initiate cDNA synthesis and the recovery of the resultant cDNAs using streptavidin-magnetic Dynabeads.

US 5,741,678 discloses a quantitative method for early detection of mutant alleles. In particular, Figure 2 discloses nucleotide sequences that are labelled with biotin and which can bind to a supporting surface coated with avidin or streptavidin.

US 5,602,300 discloses a process for detecting mutations, *inter alia.* It discloses the use of solid particles coated with a lacI repressor protein which bind to DNA molecules containing a lacZ operator sequence.

There is thus a need for a new method of at least partially separating nucleic acid molecules into different populations. The present invention provides such a method.

According to the present invention, there is provided a method for at least partially separating nucleic acid molecules in a sample into populations wherein a population is tagged with a protein capable of being immobilised on a matrix, said method comprising contacting the nucleic acid containing sample with a matrix which selectively binds proteins, whereby the said protein interacts directly with the matrix, whereby the tagged molecules are captured by the matrix and thereby separated from untagged molecules.

This method is much simpler than the aforementioned electrophoretic separation method and is also quicker. It is thereby more cost effective, has greater all round efficiency, and does not suffer from the drawbacks of electrophoretic separation.

The method relies upon the tagged nucleic acid molecules being captured, i.e. immobilised or retained on the matrix, thereby effecting a separation from untagged molecules which remain in solution.

The method of the invention can be used to separate untagged nucleic acid molecules of interest (target molecules) from tagged unwanted nucleic acid molecules or fragments, in which case it is the unwanted nucleic acid molecules which are captured by the matrix, leaving the target molecules free in solution. This is advantageous where the desired target nucleic acid molecules are intended for downstream processing for example by further genetic manipulation techniques since it enables the further steps to be carried out without the need to elute or otherwise detach the desired nucleic acid molecules from the matrix, and it also avoids the need, if necessary, to remove the tag from the nucleic acid molecules. This thus constitutes a preferred aspect of the invention. The method may however also be used to separate tagged target nucleic acid molecules of interest from untagged unwanted nucleic acid fragments, in which case it is the target molecules of interest which are captured by the matrix leaving the unwanted nucleic acid molecules in solution. Additionally, the method may be used to collect all separated nucleic acid fractions for different downstream purposes.

As used herein, 'nucleic acid molecule' refers to any nucleic acid molecule, including DNA, RNA, cDNA and hybrid compounds such as compounds comprising nucleic acids and peptides such as peptide nucleic acids (PNA); and in the case of DNA, it includes double stranded and single stranded molecules, and any synthetic DNA or RNA molecule and hybrid DNA/RNA molecules (ie molecules where one strand is DNA and the other is RNA). 'Target' or 'desired' DNA refers to the nucleic acid molecule which is intended to be isolated or separated from other nucleic acid molecules. In the context of the invention, 'tag' refers to a protein which can be attached to, bound to, incorporated in, carried by a nucleic acid molecule, or be part of the nucleotide sequence of the nucleic acid molecule, or otherwise linked to a nucleic acid molecule, and which serves as a means for capturing the tagged population of nucleic acid molecules from a nucleic acid containing sample in which one particular population is tagged in this way and other populations are not tagged. The tag thereby enables the nucleic acid mixture to be fractionated, with tagged molecules being separated from untagged molecules by means of a retention step using a matrix. The tag may be incorporated into the nucleic acid molecule, i.e. be part of the nucleic acid molecule, for example it may be part of a modified nucleotide and incorporated into the nucleic acid molecule during synthesis, or be a part of the nucleic acid sequence of the molecule, in which case the nucleic acid molecule is itself referred to as tagged, or the tag may be attached or bound to a nucleic acid molecule, for example by post synthetic steps for example by addition of terminal nucleotides, or by binding to a recognition sequence within the nucleic acid sequence, in which case the nucleic acid, without tag attached, is described as being capable of being tagged.

The method may be used to separate or fractionate any of the aforementioned classes of nucleic acid, or mixtures of these. A preferred aspect of the invention comprises the fractionation of DNA molecules or fragments in a sample to at least partially separate the sample into different populations of nucleic acid molecules. Examples of such methods includes the separation of particular restriction fragments from a restriction enzyme digested DNA preparation, for example a PCR generated DNA molecule, or a recombinant DNA molecule, and the "clean-up" of PCR reactions, i.e. the removal of PCR products which are the result of misannealing of primer. The method has other applications also, and may be used, for example, to separate linear and circular nucleic acid, in diagnostic PCR and in vitro packaging of bacteriophage.

The moiety used to tag nucleic acid molecules in accordance with the method of the invention may be any protein which is capable of tagging a nucleic acid molecule and of immobilisation on a matrix which selectively binds proteins.

The nature of the tag will depend at least in part upon the molecules which are to be separated and on the matrix used. Examples of suitable tags include proteins which can be incorporated into a nucleic acid molecule, or which can be used to modify individual nucleotides within a nucleic acid molecule, and proteins which have an affinity for a particular binding site within a nucleic acid molecule. The tag may thus be introduced into the nucleic acid molecule during its synthesis, for example by means of a labelled nucleotide, or after synthesis for example by addition at one end of labelled nucleotides e.g. by means of an enzymic reaction.

In one embodiment, the nucleic acid molecule may be immobilised on the matrix in the method of the invention by means of a protein which is a binding partner to a small molecule ligand, wherein the ligand serves as a separate linking group between the nucleic acid molecule and the protein binding partner.

In one embodiment of such a method, the nucleic acid sample is first contacted in solution with a protein binding partner for the ligand, which binds only to the ligand-tagged nucleic acid molecules, and the binding partner bound tagged nucleic acid molecules are then extracted by means of a matrix with affinity for proteins.

One example of a ligand which may be used in the invention is biotin. Others are known in the art. Where biotin is used as the ligand, the binding partner is avidin or streptavidin, and the matrix may be one which has an affinity for proteins and is thereby able to capture streptavidin or avidin and any molecules to which (strept)avidin is bound. Avidin and streptavidin may each be used as the binding partner for biotin, and where in the following reference is made to streptavidin, the bacterial protein, it will be understood that avidin could also be used. Biotin can be readily incorporated into nucleotides, and indeed biotinylated nucleotides are available commercially. We have also found that the use of a biotin ligand is very efficient in the method of the invention. Biotin accordingly represents a preferred ligand for the method of the invention.

In one embodiment where biotin is used as the ligand, biotinylated nucleic acid molecules may first be incubated in solution with streptavidin, whereby streptavidin as binding partner will bind to any biotin containing nucleic acid molecules and form a binding complex. These tagged molecules with streptavidin attached are then subsequently immobilised by means of a matrix capable of selectively immobilising proteins, whilst not being capable of immobilising nucleic acids at least under the conditions used, thereby separating out from the sample the biotin containing nucleic acid molecules.

In another embodiment, the ligand may be fluorescein or digoxigenin or an antigen. These ligands may be captured on the matrix by means of protein binding partners to these ligands, for example an antibody to the ligand, either polyclonal or monoclonal, or a fragment of such an antibody. Where the ligand is a steroid, the capturing means may be either an antibody, or a fragment thereof, or a receptor for the steroid, or a fragment thereof with steroid binding properties. These capturing means may be utilised in a similar fashion to the aforementioned use of streptavidin, with a matrix which has an affinity for proteins.

In a further embodiment of the invention, the tag is a nucleic acid binding protein, which has a specific recognition sequence within the nucleic acid molecule to be tagged. Examples of such nucleic acid binding proteins include the transcription factor AP-1 which binds to the AP-1 recognition sequence, the myb protein which binds to a specific short recognition sequence, and the lacI repressor protein, which binds to a lac operator sequence.

In such embodiments of the invention, a sample containing nucleic acid molecules which includes the protein recognition sequence may be first tagged in solution by contact with the protein recognised by the specific sequence, and then the sample is contacted with the matrix whereupon tagged molecules are retained on the matrix leaving untagged molecules i.e. molecules without bound protein in solution.

In each of these embodiments, a protein is involved in capturing a population of nucleic acid molecules, either as the tag itself, or as the binding partner for a ligand (such as antibody, or streptavidin). This is advantageous because it enables protein receptive materials to be used as the matrix, preferably materials which have selective binding for proteins and thus which do not bind nucleic acids, at least under the conditions used, thereby ensuring that untagged molecules are not captured by the membrane and sequestered from the sample. The protein may be captured by the matrix and bound to it by a variety of interactions, including ionic interaction, hydrophobic interaction and affinity binding.

The matrix may take any convenient physical form, and many are known in the art, for example sheets, gels, filters, membranes, fibres, tubes, microtitre plates, columns, particles, and may be particulate or porous. Porous materials such as filters and membranes are convenient for separation methods according to the invention, either for filtering away unwanted tagged DNA or for collection of wanted tagged DNA. Examples include samples where an untagged population is intended for further downstream processing, and where the tagged nucleic acid population, constituting the 'undesired' population, may be captured from solution by the membrane, since it is straight forward to process the sample by filtration through the membrane offering an effective and rapid capture method and simultaneously fractionating into the filtrate the untagged nucleic acid population, thus offering a straightforward route into the next manipulation stage. Porous materials such as membranes thus constitute a preferred matrix for use in the invention.

Porous matrices may thus be conveniently used for filtering away unwanted tagged nucleic acid molecules or for collection of wanted tagged nucleic acid molecules. Such matrices may be used as part of a device for a single or multistep separation, or as part of other steps, such as for detecting, assessing or quantitating DNA or any other product in a downstream reaction stream. These porous matrices may be incorporated into separation devices such as centrifuge vials, microtitre plates, cartridges or syringes, and, depending on the sample and the downstream processes to be operated, one or more of such devices may be provided in a serial manner. Such devices may be handled manually, semiautomatically or in fully automated fashion.

In one embodiment, a NycoCard™ may be used. Where the tagged nucleic acid fragment is the sequence or molecule to be detected, it may, after binding for example to a protein with affinity for the tag be entrapped directly in a protein binding membrane retained in a NycoCard device. Such a device would include an appropriate membrane with an absorbent pad such as cellulose paper placed on one side to enhance passage of the liquid sample through the membrane. In one embodiment, an impermeable sheet may be placed over the other side of the membrane and holes may be provided to permit application of samples to the membrane in the case where multiple samples are to be analysed. Where the tagged sequences are to be eliminated, and untagged nucleic acids collected, a protein binding filter may be used as a prefilter, placed over a nucleic acid binding filter mounted in the NycoCard device so that unwanted tagged molecules are retained on the prefilter, and desired untagged molecules retained on the nucleic acid binding filter.

The matrix may be composed of a variety of materials known in the art for the purpose, including polymeric materials for example cellulose, polystyrene, agarose, latex, which may be derivatized or modified to provide means for capturing the tag itself. Thus for example, the material may be treated eg by coating with a substance having an affinity for proteins. Preferably, the matrix will have specificity for proteins as compared to nucleic acids, so that untagged molecules are not retained by the matrix. In the invention, the capturing process involves a protein, either where the tag is a protein, or where a ligand which has a proteinaceous binding partner which links the ligand to the matrix, and the matrix is thus of a protein receptive nature. Examples are known in the art and include known protein binding matrices, coated for example with polymers having a specific affinity for proteins, at least under the conditions used. Particularly preferred are membranes comprised of protein binding polymers, such as those described in WO98/23630,EP-0524800 and EP-0580305, for example Centriflex (TM) marketed by Edge Biosystems, USA.

The separation method of the invention is particularly convenient where a sample is to be fractionated, and untagged nucleic acid molecules in solution are to be collected for further downstream processing. The method may however also be used where it is the tagged nucleic acid molecules which are to be collected for further processing. When tagged molecules are those to be collected for downstream work, the molecules will need to be released from the matrix, and, depending on the capture method used, may also need to be released from the binding partner for the ligand. The release method used may depend upon the nature of the ligand, and its binding partner, and the type and strength of their mutual binding forces.

The reaction conditions chosen for the release step may also be selected so as to prevent the released tagged nucleic acid from rebinding to the matrix or to its binding partner. Examples of such methods are known in the art. Thus for example, chemical or physical conditions may be changed so as to aid release of tagged nucleic acid molecules from the matrix -bound complex. Examples of chemical methods include altering ionic strength or pH, addition of chelating agents, and the use of competing free tag molecules, or molecules chemically related thereto, molecules comprising tags or tag like moieties, molecules or ions which change the conformation of the binding partner and thereby reduce, eliminate or modify the ligand-binding partner interaction, addition of detergents or dissociating agents, or by enzymatic treatment. Examples of physical methods include changes in temperature, sonication, vibration. Any combination of these physical and chemical methods may be used.

Depending on the strength of the interaction between the ligand and its binding partner, it may be possible to release the ligand or the tagged molecule from the binding partner or matrix by means of adding excess ligand which competes with the ligand-tagged DNA for attachment sites to the binding partner for the ligand or to the matrix, and the released tagged DNA may then simply be washed off. Thus where the ligand is fluorescein, and the binding partner is an antibody to fluorescein, fluorescein-DNA may be released from the matrix by addition of free fluorescein in solution. Where however the ligand-binding partner interaction, binding partner- matrix interaction or tag-matrix interaction is strong, addition of free tag is not always effective at disrupting the interaction. In this case, other methods such as by degrading the binding partner for example by means of pH or enzymes in such a way that it is released from the matrix and/or the ligand may be used. Release may also be effected by disruption of the matrix into a form unable to bind proteins, such as by means of chemical agents, so that the protein-DNA or protein-ligand-DNA complex is released, or by chemical means which disrupt the interaction or otherwise effect the affinity between protein and matrix.

The biotin-streptavidin binding pair is one where there is a particularly strong interaction and thus is not susceptible to disruption by means of addition of free biotin. Thus where the ligand is biotin, and the ligand-tagged DNA is captured on the matrix by means of avidin or streptavidin, biotinylated DNA cannot be released from the membrane by addition of free biotin. This method can however be used where the ligand is a biotin derivative with lower binding affinity than biotin to streptavidin, in which case the ligand-tagged DNA may be released from the matrix by addition of free biotin. This may compete with the biotinylated DNA for attachment sites to streptavidin and the released biotinylated DNA may simply be washed off the matrix.

Depending on the ligand used, it may be necessary to incorporate a step whereby the tagged DNA which is intended for downstream processing is released from the binding partner for the ligand which serves to immobilise the ligand-tagged DNA on the matrix. Examples include addition of chemical substances or ions or by applying physical conditions capable of reducing the binding force between the ligand and its binding partner and then collecting the released DNA.

The method of the invention can be used in a number of different applications, including analytical, preparatory and diagnostic uses, examples of which are presented below. Other applications will be apparent to those skilled in the art.

An important application of the invention is in the cutting and ligating of DNA molecules, such as in the separation of particular restriction enzyme digestion products, and the separation of ligated, circular DNA molecules from other products of ligation reactions.

Thus one application of the invention is in the manipulation of restriction enzyme digested fragments of DNA particularly where a specific fragment is required for further manipulation which thus needs to be separated from other products of the enzyme reaction. The separation method enables a population of linear DNA molecules which are tagged at one or both ends to be separated from untagged molecules. The molecules may be labelled during synthesis, for example using labelled nucleotides such as biotinylated nucleotides, e.g. in the form of biotinylated primers, or the molecules may be labelled enzymically by end-labelling methods known in the art.

Thus viewed from a further aspect, the present invention provides a method for at least partially separating a mixture of restriction enzyme digested fragments of DNA wherein the starting material is a linear DNA molecule which is tagged or capable of being tagged at or near one or both ends with a protein capable of being immobilised on a matrix, said method comprising subjecting the DNA molecule to restriction enzyme digestion followed by contacting the sample with a matrix which selectively binds proteins whereby the tagged molecules which originate from an end of the starting material are captured by the matrix and are thereby separated from untagged molecules.

This method is particularly well suited to separating digestion products of PCR produced DNA because of the way the synthesis works. In the PCR method of DNA amplification, two specific oligonucleotide primers are used, one of which is complementary to and therefore hybridises to the 5' end of the coding strand and one of which is complementary to and therefore hybridises to the 5' end of the noncoding strand so that in the presence of appropriate DNA polymerase enzymes, a full length copy of the target sequence can be synthesised. This copy will have the primer oligonucleotide incorporated into each of the two strands of the synthesised DNA, at the 5' end of each strand. Such PCR synthetic methods are often used to prepare specific DNA molecules or fragments for subsequent genetic manipulation, where the specific fragment of interest can be obtained by restriction enzyme cutting of a longer PCR product. As previously mentioned, these subsequent manipulations are often inefficient because of the presence in the subsequent steps such as ligation mixtures of other products from the restriction enzyme digest such as partially digested products and undigested DNA molecules. In circumstances where it is an internal fragment of the full length PCR product which is the product of interest for downstream processing, the 'unwanted' by-products of the restriction enzyme digest will include at least one terminus of the full length PCR product, the method of the invention can be used to effect an at least partial separation of the product of interest from end-containing fragments, and undigested molecules, by utilising labelled primers for example biotinylated primers. In this way, tagged nucleic acid molecules can be removed from a nucleic acid sample, leaving in solution a sample containing the desired internal restriction fragment which, since it does not include a terminus for example incorporating a PCR primer, will not be tagged.

A further aspect of the invention pertinent to PCR methodology is in the so-called "clean up" of the products of the PCR reaction, i.e. the removal of unwanted products which include products which are the result of misannealing of the primers. The larger the nucleic acid template is, the more this can be a problem. This method has utility where there are unique restriction enzyme sites or other cleavable sequences, at or close to the ends of the nucleic acid sample to be amplified in the PCR reaction.

The method involves the use of PCR primers which are tagged, or capable of being tagged with a protein, and which are complementary to the ends of the sequence to be amplified and thus are capable of hybridising to the template nucleic acid and which also overlap only partially with each of the unique restriction sites. With the use of primers which are complementary to and hybridise to the full restriction enzyme recognition. sequence within the template nucleic acid, every nucleic acid molecule produced by the PCR reaction will carry the restriction enzyme recognition site irrespective of whether or not the primer has annealed to the desired sequence or whether the PCR product is the result of misannealing of the primer. However, using primers which hybridise to only part of a restriction enzyme recognition site in the template, it is only in the PCR products which derive from the intended annealing where the restriction enzyme recognition site is reconstituted in the PCR product. Thus by using tagged primers, or primers which are capable of being tagged, it is possible to clean up PCR reaction products by performing the PCR reaction, and subsequently cleaving or digesting the PCR reaction products with the particular restriction enzymes specific for the said unique restriction recognition sites. Upon such restriction enzyme digestion, it is only the correct PCR products which are cleaved and which, because cleavage resuts in removal of the primer and tag, can be separated from the unwanted PCR extension products which, as they are not cut by the restriction enzymes, still retain the tag.

Thus viewed from one aspect, the present invention provides a method for at least partially separating the correct and desired products of PCR amplification from PCR products which result from incorrect annealing of a PCR primer to template, wherein the template nucleic acid molecule comprises a unique restriction enzyme recognition site at or towards an end of the template, and a PCR primer which is tagged or capable of being tagged with a protein is complementary to a sequence on the template which extends partially into the unique restriction site, the method comprising amplifying the template by means of PCR, digesting the PCR products with the restriction enzyme specific for the said unique restriction enzyme recognition site, and contacting the resulting product with a matrix capable of binding proteins whereby tagged nucleic acid molecules are captured by the matrix and thereby separated from untagged molecules.

In this context, unique restriction enzyme site refers to restriction enzymes which have only a single recognition site within the template nucleic acid molecule, but also includes the special case where the same restriction site may be present at or towards each end of the template.

In one embodiment, the template nucleic acid molecule to be amplified by PCR incorporates only a single unique restriction site at or near only one of the ends. When the reaction products are treated with this enzyme this results in a partial "clean-up" of the PCR products the degree of which is dependent at least in part upon the extent to which the primer which anneals to the region of the template which includes this unique site is responsible for incorrect annealing. In this embodiment, it is the primer which extends into the unique restriction enzyme site which will be tagged or capable of being tagged. In a preferred embodiment, the template to be amplified by PCR incorporates a unique restriction enzyme site at or near to each end. These sites may be sites for the same restriction enzyme, i.e. a restriction enzyme which cuts the template and the PCR copies twice, once at each end, or they may be sites for different restriction enzymes, provided that each of said enzymes has only a single recognition site within the template, and that the two sites are one at each end of the template.

In this method, the location of the or each unique restriction site in relation to the terminus of the nucleic acid template depends, at least in part, upon the particular restriction enzyme, since different enzymes have different minimum distances from the terminus for efficient restriction cutting. These preferences are known in the art, and are described for example in the catalogues of manufacturers of restriction enzymes. In general, the restriction site will be at least one base from the terminus. The choice of enzyme and its location may be readily determined by the skilled person based on knowledge in the art and according to manufacturer's information. In general, the restriction site will be located at least the minimum distance from the terminus for efficient cutting by the corresponding enzyme, although the site can be further away from the terminus. Examples of this minimum distances are at least one base from the terminus for BamHI and at least six bases from the terminus for NdeI. Minimum distances may be ascertained from the published catalogues of suppliers of restriction enzymes, for example the New England Biolabs Catalogue 1999, and from Moreira and Noren, Biotechiques 19, 56-59 (1999).

Thus by using or constructing by means of recombinant DNA techniques known in the art a template comprising a unique restriction enzyme site at or near each end, it is possible to remove the unwanted PCR by products which are the result of mismatched annealing of PCR primers at locations on the template other than those intended. Where both primers are tagged or capable of being tagged, all PCR products will initially be tagged at both ends. By subjecting the PCR products to restriction enzyme digestion with the aforementioned restriction enzymes which have unique recognition sites towards each end of the template, either simultaneously or sequentially, molecules which are uncut, or cut at only one end will retain the tag, as will the termini from the correct PCR product which have been created by the restriction enzyme cutting; these may be separated from the desired product which will have had both primers removed and thus no longer contain a tag or a moiety capable of being tagged. This has the advantage that expenditure of large amounts of time and materials to fine-tune the PCR reaction is avoided, as is the need for gel electrophoresis or other methods of separating PCR products by size. An at least partial "clean up" can however be achieved if only one of the two PCR primers is tagged, or capable of being tagged.

Conveniently, the ligand may be biotin, in which case the PCR products, before or after restriction enzyme digestion are contacted with streptavidin, and then the tagged molecules with streptavidin bound are separated by protein-binding matrices from untagged molecules.

In this particular embodiment of the invention, the tag may be attached at any position on the primer, so long as the 3' end of the primer is available for elongation by the PCR polymerase. Conveniently, the tag may be attached at the 5' end of the primer, since adding a tag at this position does not pose synthetic difficulties. In addition, without wishing to be bound by theory it is believed that a tag placed at the 5' end of the primer will be less likely to interfere with the activity of the PCR polymerase, and also, that it may be better situated for capture in any of the capture methods described herein.

An example is shown below:

5'-tttactggatcctag------ttacgtacattaatcgg-3'

3'-aaatgacctaggatc------aatgcatgtaattagcc-5'

This fragment has two unique restriction sites, one for BamHI at the "left" (ggatcc) and one for AsnI at the "right" (attaat). To amplify this fragment in the usual manner, two primers are usually made, optionally labelled with biotins at their 5'-ends, as illustrated below:

BamHI primer: 5'-biotin-tttactggatcctag-3'

AsnI primer:    5'-biotin-ccgattaatgtacgtaa-3'

Using these primers in a PCR reaction will typically produce a number of products, mainly due to misannealing of the primers during PCR.

The BamHI and AsnI primers fully implement the nucleic recognition sequences in their sequences. That has the consequence that every DNA fragment produced in the PCR reaction by these primers has the BamHI and the AsnI restriction enzyme recognition site. All fragments produced in the PCR reaction will be able to be cut by the BamHI and the AsnI enzyme, both the correct (ie desired) ones and by-products.

However, by the use of slightly shorter primers in this embodiment of our invention, we can very efficiently remove all unwanted by-products.

Example of primers which can be used are:

BamHI primer short: 5'-biotin-tttactgga-3'

AsnI primer short: 5'-biotin-ccgatt-3'

These primers only partly overlap the recognition sites for the restriction enzymes. Therefore, only by annealing to their intended nucleic sites, they will form complete enzyme recognition sites. As a consequence, only correct (or desired) fragments produced from the PCR reaction are able to be cut by both restriction enzymes.

The four possible groups of PCR products which will result by the use of the primers (B=biotin label) are as follows:

5'-Btttactggatcctag------ttacgtacattaatcgg-3'
3'- aaatgacctaggatc------aatgcatgtaattagccB-5'
(Correct fragment - two restriction sites)

5'-Btttactggtag------ttacgtacattaatcgg-3'
3'- aaatgaccatc------aatgcatgtaattagccB-5'
(Incorrect fragment - only one restriction site (AsnI))

5'-Btttactggatcctag------ttacgtacaatcgg-3'
3'- aaatgacctaggatc------aatgcatgttagccB-5'
(Incorrect fragment - only one restriction site (BamHI)

5'-Btttactggatag------ttacgtacaatcgg-3'
3'- aaatgacctatc------aatgcatgttagccB-5'
(Incorrect fragment - No restriction sites)

Cutting by AsnI and BamHI gives the following fragments:

5'-Btttactg    gatcctag------ttacgtaca    ttaatcgg-3'
3'- aaatgacctag    gatc------aatgcatgtaatt    agccB-5'
(Correct fragment - two restriction sites- both biotins
removed)

5'-Btttactggtag------ttacgtaca     ttaatcgg-3'
3'- aaatgaccatc------aatgcatgtaatt     agccB-5'
(Incorrect fragment - only one restriction site (AsnI)
only one biotin removed )

5'-Btttactg     gatcctag------ttacgtacaatcgg-3'
3'- aaatgacctag     gatc------aatgcatgttagccB-5'
(Incorrect fragment - only one restriction site (BamHI)-
only one biotin removed)

5'-Btttactggatag------ttacgtacaatcgg-3'
3'- aaatgacctatc------aatgcatgttagccB-5'
(Incorrect fragment - No restriction sites - no biotins
removed due to no restriction)

In a mixture of all the fragments mentioned above, only one fragment, the correct one, will be without biotin attached. By adding streptavidin to the fragments, and contacting the reaction products with a protein binding matrix, for example by passage through a centriflex membrane, only the correct fragment will not be sequestered, and , in the case of a centriflex membrane, will pass the membrane unhindered.

The difference between using the short primers (ie primers which do not completely extend into the restriction enzyme site on the template) and the long primers (which incorporate the complete restriction enzyme site) is evident, since, in the case of long primers, all fragments, even the erroneous ones, would have the restriction-sites for AsnI and BamHI introduced. In that case, it would not be possible to distinguish between correct and erroneous products of the PCR reaction.

The method may also be used to separate restriction enzyme digestion products of DNA molecules which are either linear or have been linearised and which are tagged at one or both ends by means of end-labelling methods such as those which are known in the art, for example by way of enzymes. One example is the enzyme terminal deoxynucleotidyltransferase (TdT) which can be used alone or together with DNA polymerase I (Klenow fragment) to add tagged nucleotides, for example biotinylated or fluorescein or digoxygenin labelled nucleotides to the free hydroxyl groups at the 3' ends of a linear DNA molecule, thereby adding a ligand to the 3' ends. In the case where the linear DNA to be subjected to restriction enzyme digestion is blunt ended or has protruding 3' ends, then only TdT is needed to tag the 3' ends. where however the 5' end is protruding, and the 3' end is recessed, an enzyme such as Klenow fragment is additionally needed to fill in the recessed end to enhance efficiency.

Where the ligand is biotin, the sample may first be contacted with streptavidin in solution, and then the sample is passed through a protein binding membrane which binds to streptavidin and thereby retains the unwanted, tagged, nucleic acid molecules, leaving the desired fragment in solution and in a form suitable for further manipulation.

This represents a significant advance over current procedures which are labourious and involve the separation of the restriction enzyme digestion mixture on an agarose gel, visualising the fragments by ethidium bromide staining, excising the DNA fragment of interest and purifying it from the gel which is a much more lengthy procedure and because of this involves a greater risk of exposure of the DNA to nuclease activity.

A further application of this aspect of the invention is in the field of restriction enzyme digestion of PCR amplified products. Thus there are circumstances where it is desirable to carry out partial restriction enzyme digestion, ie with limited amounts of enzyme. Restriction enzyme preparations often contain small amounts of endonuclease which can degrade the ends of newly cut DNA, which can reduce the quality of the fragment of interest, and cause difficulties in further manipulation steps such as ligating the DNA, for example into an expression vector. Such problems caused by contaminating nucleases can however be minimised by using limited amounts of restriction enzyme and reducing the incubation periods. A drawback however is that the resulting digest will include unwanted byproducts of the restriction enzyme digestion, such as only partially cut molecules or uncut molecules. The method of the invention however enables partial digestion to be exploited, by synthesising the substrate for restriction enzyme digestion by PCR using labelled eg biotinylated primer DNA so that any DNA molecule or fragment which includes at least one terminus (such as undigested molecules, or partially digested molecules) can be removed or sequestered by means of an appropriate matrix, leaving a solution enriched in the internal restriction fragment of interest.

A further application of the tag-based separation method of the invention is in diagnostic PCR. The use of PCR for detecting mutations including mutations which differ by as little as only a single nucleotide such as occurs in sickle cell disease is known. In some cases, the size of a PCR fragment will be indicative of the presence or absence of a particular mutation. Usually, however, diagnostic PCR techniques generally require further techniques following the initial PCR on the patient sample in order to diagnose whether or not the mutation is present in the sample, for example restriction analysis and/or sequencing. These can be costly since they often require expensive chemicals such as peptide nucleic acids and also time consuming, contributing to a delay in ascertaining the medical status of the patient. The existing methods are considerably simplified by using the tag-capture method of the invention by means of PCR reactions using at least one PCR primer which is tagged, or capable of being tagged. By using tagged primers no such further steps are needed, and the presence of a mutation can be detected using the PCR amplification step alone.

Thus the method of the invention when applied to diagnostic PCR is able to replace the need to sequence PCR products in accordance with current diagnostic methods. In this embodiment, the primer for annealing to and extending the 3' end of the sample is tagged, or capable of being tagged in such a way that it retains a 3' OH group and can thus be elongated in a PCR reaction, and is specific for the mutant nucleic acid; in other words, it hybridises to the sequence in the nucleic acid which is altered in the disease state. Alternatively, the 3' primer may hybridise to the normal nucleic acid sequence.

In one embodiment, the method may be used to detect single base mutations in which case the 3' end of the primer corresponds to the mutated base. In the case of multibase mutations, the 3' end of the primer may correspond to any one of the mutated bases.

Thus, where the mutation is known, in one version of this embodiment, two PCR reactions are carried out on the sample DNA, each uses the same primer for the 5' end of the sample, but with a different 3' primer, one hybridising to and capable of producing an extension product corresponding to the 'normal' DNA, and one complementary to and specific for the mutant nucleic acid sequence and thus capable of producing an extension product corresponding to the mutant sequence. In the case of PCR of a mutated target using the tagged primer appropriate for the mutated DNA, PCR amplification products will incorporate the tag whilst in the PCR reaction using the 'normal' primer there will be a mismatched base at the 3' end of the primer which will be removed by the PCR polymerase, such as Tag polymerase, and, if the 3' base is tagged, its removal will remote the tag or the moiety capable of being tagged which it bears. The PCR product will thus not incorporate the tag. Thus it is only the PCR reaction products of the mutant nucleic acid which are formed by extending the mutant primer which are tagged, or capable of being tagged, and which can be detected using any of the aforementioned methods.

The converse situation arises in the case of PCR extension of 'normal' DNA which does not contain the specific mutation to be detected. In this case, it is the PCR products obtained using the mutant primer where the 3' nucleotide of the primer is mismatched and accordingly removed together with the tag which cannot thus be detected in the full length reaction product whilst the product using the 'normal' primer will include the tag or the moiety capable of being tagged which can then be detected.

In this embodiment, the tag or moiety capable of being tagged is on or is attached on or to the 3' base, in such a way that the 3' OH is still extendable by PCR polymerase.

Thus viewed from a further aspect, the present invention provides a method of diagnostic PCR in which a test sample is separately subjected to PCR reactions, in which the mutation, if present, is in the sequence to which the 3' primer is complementary, a first PCR reaction using a 3' primer complementary to the normal target nucleic acid and a second PCR reaction using a 3' primer complementary to the mutant target, wherein the 3' nucleotide of the mutant primer corresponds to one of the nucleotides which is mutated in the mutant nucleic acid, each of said 3' primers bearing a tag or being capable of being tagged on the 3' nucleotide of the primer, wherein the presence or absence of the tag in the PCR reaction products is detected.

In its simplest embodiment, the 3' primers are labelled, for example with biotin, which may be detected using any of the methods described herein. Examples of methods of detecting biotinylated PCR products include the addition of streptavidin and filtration through a protein binding membrane where retained DNA is detected, and passage through a membrane such as a nitrocellulose membrane, for example as in the aforementioned NycoCard™ system. In an alternative method, streptavidin may be added to the PCR reaction products and the mixture passed through a Centriflex membrane. If no DNA is detectable in the material passing through the membrane, the DNA will have been retained on the membrane, and thus will have been biotinylated.

In circumstances where the primer is not itself tagged, but is capable of being tagged, the tag will need to be added prior to the detection step.

In an alternative embodiment, a single PCR reaction may be carried out using a tagged primer specific for either of the normal or mutated sequences, with the presence of the tag in the PCR products being indicative of the presence or absence of the mutation, depending upon whether the primer is specific for the normal or the mutant DNA. Thus if the tagged primer is specific for the normal DNA, the PCR product with normal DNA will be tagged, whilst that with the mutant DNA will not, and if the tagged primer is specific for the mutant DNA, the PCR product on normal DNA will be untagged whilst the products of PCR with a mutant DNA will be tagged and thus detectable. Thus whilst carrying out duplicate PCR reactions gives more certainty, a single PCR at least gives an indication of the presence or absence of the mutation.

Thus viewed from a further aspect, the present invention provides a method of diagnostic PCR of a mutation in a nucleic acid molecule in which the presence or absence of a mutation in a nucleic acid sample is detected, wherein a 3' primer specific for either the normal or mutant nucleic acid is used, wherein said primer is complementary to a region of the nucleic acid where there is a base difference between the normal and mutated DNA with the 3' terminus of the primer corresponding to the position in the sample where there is a difference between the normal and mutant, the primer being tagged or capable of being tagged, whereby the presence or absence of the tag in the PCR product is detected.

It is also possible to carry out at least a preliminary diagnosis using a 3' primer which is specific for the normal DNA.

The present invention accordingly provides from a further viewpoint a method of diagnostic PCR in which a test sample is subjected to PCR with primers complementary to the normal target DNA wherein the primer for extending at the 3' end of the target anneals to a sequence the 3' nucleotide of which is mutated in the mutant, the 3' primer bearing a tag or being capable of being tagged at or on the 3' nucleotide, wherein the presence of the tag in the PCR reaction product as detected.

In this way, the absence of a detectable tag in the product indicates that the sample does not contain normal DNA.

An example of detecting a single base mutation is as follows:

In this case, the 3' end of the primer for extending the 3' end of the sample aligns with the mutated DNA on the template.

Assume the case where it is to be determined whether a patient has a normal or an abnormal genetic mutation.

A normal DNA sequence of the gene in question is:

5'-ccccatg---------atgacctaggAccacct-3'

3'-ggggtac---------tactggatccTggtgga-5'

The patient's DNA looks like this:

5'-ccccatg---------atgacctaggCccacct-3'

3'-ggggtac---------tactggatccGggtgga-5'

A mutation is present. The knowledge of the patient's DNA is not necessary to do the test. Only the normal condition needs to be known in order to diagnose the mutation.

Primers are needed in order to do PCR, one primer annealing in the 5'-end area, and one in 3'-end area. The 5'-end primer will be identical for both patients with or without the mutation, as follows: No1-primer: 5'-ccccatg-3' For the other 3'-area, a primer matching the normal situation would be needed as follows: No2normal: 5'-aggtggt-3' If the mutation is known, a primer may be constructed for this, as follows:

No2abnormal: 5'-aggtggg-3'

In order for the method to work, the last 3'-end nucleotide is labelled with a biotin, still leaving it able to elongate in a PCR reaction (having a free 3'-end OH-group)
The 3'-end primers will now be like this: No2normal: 5'-aggtggtB-3' 
No2abnormal: 5'-aggtgggB-3'
(B=biotin)
 The PCR products obtained from using these primers on normal DNA will be:

(No1+No2Normal primers)
5'-ccccatg----------atgacctaggAccacct-3'
3'-ggggtac----------tactggatccTggtgga-5'
|
biotin

(No1+No2abnormal primers)
5'-ccccatg---------atgacctaggAccacct-3'
3'-ggggtac---------tactggatccTggtgga-5'  +  biotin

Only use of the No2normal primer on normal template DNA will give biotinylated PCR products. PCR enzymes will remove the biotin from the No2abnormal primer, giving an unbiotinylated product.

The PCR products obtained from using these primers on abnormal DNA will be: 
(No1+No2abnormal primers)
5'-ccccatg----------atgacctaggCccacct-3'
3'-ggggtac----------tactggatccGggtgga-5'
                              |
                              biotin
 
(No1+No2Normal primers)
5'-ccccatg----------atgacctaggCccacct-3'
3'-ggggtac----------tactggatccGggtgga-5'
 As above, but just the opposite.

The presence of biotinylated product will to show whether or not a patient has a mutation.

A specific example of diagnostic PCR of sickle cell anaemia caused by a single base mutation is as follows: In this example, the primer is indicated as being labelled with biotin. This is one example of a ligand which may be used and is not to be construed as limiting this illustrative example; other ligands may be used.

Normal human hemoglobin A1 has a DNA sequence for the first exon (coding part) of the haplotype A1 beta-globin gene beginning as shown:

### Normal DNA:

atg gtg cac ctg act cct gag gag aag tct gcc gtt act gcc
ctg tgg ggc aag gtg aac gtg gat gaa gtt ggt ggt gag gcc
ctg ggc agg ...

which translates to the

### Normal aminoacid sequence:

MVHLTPEEKSAVTALWGKVNVDEVGGEALG...

For the human disease sickle cell anaemia, the DNA sequence for the first exon of haplotype S beta-globin gene begins as shown:

### Sickle cell disease DNA:

atg gtg cac ctg act cct gtg gag aag tct gcc gtt act gcc
ctg tgg ggc aag gtg aac gtg gat gaa gtt ggt ggt gag gcc
ctg ggc agg ...

which translates to the

### Sickle cell disease aminoacid sequence:

MVHLTPVEKSAVTALWGKVNVDEVGGEALG...

Comparison of normal and sickle cell disease DNA reveals that a single base substitution is sufficient to transform the normal hemoglobin gene to an abnormal hemoglobin gene, known to causing the serious condition of sickle cell anaemia. The gag codon is mutated to a gtg codon causing a replacement of the acidic aminoacid glutamic acid with the nonpolar aminoacid valine.

To detect the presence or absence of this mutation, genomic DNA is isolated, and, if necessary, amplified by PCR prior to diagnostic PCR in the conventional manner.

The primers used for amplification of part of the hemoglobin beta chain gene are based on the DNA sequence given by EMBEL search program when searching on the unique identifier EMBL-ID:HSBETGLOB'

The hemoglobin beta chain gene (shown below as separated triplets) is preceded by an intron sequence (intron 1) and followed by another sequence (intron 2), both introns are indicated below in italics.

Part of the introns (underlined) are used for construction of primers (see below).

gcataaaagtcagggcagagccatctattgcttacatttgcttctgacacaactgt
gttcactagcaacctcaaacagacacc atg gtg cac ctg act cct gag
gag aag tct gcc gtt act gcc ctg tgg ggc aag gtg aac gtg
gat gaa gtt ggt ggt gag gcc ctg ggc agg
ggcaggttggtatcaaggttacaagacaggtttaaggagaccaatagaaactgggc
atgtggagacagagaagactcttgggtttctgataggcactgactctctctgccta
ttggtctattttcccacc...

The non-coding introns are flanking the coding portion of the gene; the second intron separating the coding sequence from the next part of the coding sequence further downstream.

To amplify the first coding part of the gene for later diagnostic PCR, two primers may be used, for example, annealing to introns 1 and 2, respectively. Annealing sequences for the primers are indicated as *underlined italics* above.

Primer 1: 5'-ctagcaacctcaaacagacacc-3'

Primer 2: 5' -gtaaccttgataccaacctgcc-3'

The primers are only used in the amplifying PCR to gain more DNA template for the diagnostic PCR procedure, and thus need not be biotinylated or modified in any way.

For the diagnostic PCR since the nature of the mutation is known, a single base mutation in codon 6 of the exon, three primers are designed, corresponding to normal and abnormal (Sickle cell anaemia) DNA:

### Primer corresponding to Normal hemoglobin:

PrimerN: 5'-atg gtg cac ctg act cct ga-biotin-OH

### Primer corresponding to the Sickle cell disease hemoglobin:

PrimerS:   5'-atg gtg cac ctg act cct gt-biotin-OH

### Primer for the other end of the gene

Primer2: 5'-gtaaccttgataccaacctgcc-3'

(This may be the same primer as used in the amplification step; it is not tagged or modified)
Then the diagnostic test is performed with primers of step 1 and the sample or the PCR amplified sample.

Two PCR reactions are performed
a) PCR using the PrimerN + Primer 2:
b) PCR using primers + Primer2:

The PCR products from PCR reactions a) and b) are then examined for presence of biotin as previously described.

The results from examining the two PCR reactions a) and b) can have one of four possible outcomes illustrated below.

| Biotinylated PCR product or not | | Interpretation (diagnosis) |
|---|---|---|
| PCR # a (primerN+primer2) | PCR # b (primerS+primer2) | |
| Yes | Yes | Both normal and Sickle cell variants present ("harmless" heterozygous condition) |
| No | No | No normal gene present (unknown mutation in codon 6; may represent another disease?) |
| Yes | No | Only normal gene present (no mutation in codon 6; healthy subject) |
| No | Yes | Only mutated variant (Sickle cell)in codon 6 (possible lethal homozygous condition) |

The method may also be used for diagnosis of multiple base mutations, as illustrated below, again in the case of sickle cell anaemia.

A multibase mutation (bold underlined)in the first exon of beta-globin gene is shown in the DNA sequence as shown: 
atg gtg cac ctg act cct aac gag aag tct gcc gtt act gcc
ctg tgg ggc aag gtg aac gtg gat gaa gtt ggt ggt gag gcc
ctg ggc agg ...
 which translates to the aminoacid sequence:

MVHLTPNEKSAVTALWGKVNVDEVGGEALG...

A primer such as PrimerN referred to above may be used to reveal whether the normal gene is present. To diagnose the potential presence of a mutation requires a primer capable of detecting the mutation.

One primer which may be used will correspond to mutated hemoglobin as follows:

5'-atggtgcacctgactcctaac-biotin-OH

This primer would identify exactly the mutation 'acc' in codon 6.

Another primer which may be used is

5'-atggtgcacctgactccta-biotin-OH

Such a primer may be used to identify all mutations in codon 6 starting with an 'a' base.

PCR reactions are performed with the normal PrimerN, and with one or more primers corresponding to (i.e. complementary to) the mutated sequence.

This method may be used for any mutation in a known sequence of the normal gene and the hemoglobin Sickle cell mutation is given as one example. Where the common mutations seen in the population are known, a number of primers may be used to pinpoint exactly the mutation that is present.

Another utility of the method of the invention is in excising and separating DNA fragments of interest from a recombinant molecule for further manipulation. A related aspect is in excising and separating vector DNA for use in further cloning manipulations. There is a need for efficient methods of obtaining high quality linearised vector DNA fragments to be used in cloning or other biotechnological procedures. As is known in the art, vectors such as plasmids and viruses comprise in addition to appropriate elements for controlling replication and transcription one or more cloning sites for incorporation of heterologous DNA for propagation or expression. Just as restriction enzymes are used to insert a heterologous fragment into a cloning vector to prepare a recombinant vector, restriction enzymes are also used to excise the heterologous fragment for further genetic manipulation, or to excise the vector element for further manipulation. Such vectors comprise circular DNA molecules. These include a stuffer fragment, often a polylinker, and a fragment which includes the aforementioned control sequences. In one method, a recombinant vector having an inserted heterologous DNA fragment is digested with a restriction enzyme in order to excise the inserted DNA fragment which results in a mixture of linear DNA molecules, including the stuffer fragment of the vector itself, the insert, and also partially cut recombinant DNA molecules. The method of the invention may be used where the stuffer (or vector) element in the recombinant DNA molecule from which the heterologous insert is to be excised includes a specific protein recognition sequence, such as an AP-1 recognition sequence. In the method of the invention, the products of the restriction enzyme digestion reaction are contacted in solution with the protein for which the nucleic acid is specific, AP-1 in this case, and then passed through a protein-selective membrane which will sequester those nucleic acid molecules to which AP-1 has bound, leaving in solution only those DNA molecules which comprise the heterologous inserted DNA and thus do not have an AP-1 recognition sequence.

In another related embodiment, the method can be used to separate the linearised form of the vector itself for further manipulation, ie a vector which has been linearised by means of restriction enzyme digestion and from which the stuffer fragment has been excised. Such a vector fragment may then be used for inserting and ligating heterologous DNA, which, following circularisation, may be used for further downstream applications such as for transformation of host cells.

In one example, the stuffer fragment is or includes a polylinker consisting of unique restriction enzyme recognition sites. The vector is cut with one of these enzymes which will result in the molecule being linearised. The linear molecule may then be end tagged using the aforementioned enzymatic techniques. Preferably for ease of further manipulation the unique cutting enzyme is one which creates a 3' overhang. In this case, the linear vector molecule may be end tagged by the addition of labelled nucleotides such as biotinylated nucleotides by means of TdT. If the enzyme creates a 5' overhang, then Klenow fragment will additionally be needed to extend the 3' termini. We have found this method to be efficient, particularly where larger quantities of DNA are involved, greater than 10 µg. Having tagged the linearised vector, this is then subjected to further restriction enzyme digestion with one or more restriction enzymes preferably enzymes which have unique sites within the stuffer fragment one on either side of the original cutting site. In this way, end fragments are labelled and the desired fragment which is unlabelled may be separated by means of the method of the invention from the stuffer fragment. Thus when the sample is passed through the filter, fully cut vector will pass through the membrane.

In a further application, the method of the invention can be used to remove non-productive ligation products from a ligation mixture. The covalent joining of two DNA fragments using the enzyme DNA ligase is central to biotechnology. In general, ligation reactions involve insertion of small DNA fragments or inserts into a larger vector DNA. It is important that the final ligation product is correctly circularised to avoid degradation in transformed host cells, such as bacterial host cells. Thus linear ligation products are of no utility and will not survive in the host bacteria. Unfortunately, ligase reactions are not efficient, resulting typically in over 80% of linear products. These lower the efficiency of the uptake of circularised, productive ligation products in *E*. *coli.* From this point of view, the linear products can thus be considered to be contaminants or byproducts of the ligation reaction. The efficiency of transformation with ligated products could thus be enhanced if it were possible to remove the undesired linear ligation products from the reaction mixture prior to transformation. Currently there is no method available. Due to the polymorphic nature of the circular productive ligation products, these cannot be recovered by excision from gels. The present invention provides such a method.

Thus according to a further aspect, this invention provides a method of separating linear from circular nucleic acid molecules in a sample said method comprising introducing a tag to an end of a linear nucleic acid molecule, wherein said tag is a protein which is capable of being immobilised on a matrix, by direct interaction with the matrix, and contacting the sample with a matrix which selectively binds proteins, whereby said tagged linear nucleic acid molecules are immobilised on the matrix.

The method of the invention is applicable in this case because all DNA molecules present in the ligation mixture other than the desired circular molecules have free ends with exposed and reactive phosphate and hydroxyl groups. These can be end labelled by the aforementioned enzymatic methods with labelled nucleotides such as biotinylated nucleotides. Circular molecules cannot be labelled because they have no reactive 3' hydroxyl groups to which a label could be attached. In this way, tagged, linear molecules may be captured by the matrix and thereby separated from the ligated circularised molecules which remain in solution and can be used for further downstream processes, such as transformation of host cells.

In a further embodiment of the invention, the method can be used in in vitro packaging of bacteriophage particles, such as phage lambda, for example in the construction of gene libraries, and phage display libraries. Bacteriophage can be packaged either in vitro, in which case viral DNA is mixed together with the various virus coat protein components in vitro, whereupon virus assembly occurs, or in bacteria, in which case the viral DNA is introduced into appropriate bacteria which provide the necessary protein components needed for virus assembly. *In vitro* packaging is quicker than the bacterial transformation method, in that packaging can be achieved within a matter of minutes, as opposed to 1-2 days. However, it is a less efficient method than the bacterial transformation method, particularly so in the case of packaging of phage DNA which has been manipulated, for example λ DNA into which heterologous DNA has been inserted, where there are experimental indications that efficiency of packaging can be reduced by as much as 10³/µg DNA when comparing linear DNA resulting from molecular manipulations with unmodified linear lambda virus. This reduced efficiency arises, at least in part, from the presence of by products of the DNA manipulation reactions. It would thus be advantageous if these by products could be removed prior to packaging, thereby increasing the efficiency of in vitro packaging and enabling this method to be used instead of the bacterial transformation method, allowing a speedier and thus more efficient overall process. The method of the invention makes this possible, by using the tag system to tag the unwanted fragments, enabling them to be separated from the desired ligation products.

The method takes advantage of the presence of cos sites on the phage genome. These are single stranded complementary regions of DNA one at each end of the lambda genome which, upon insertion of the linear λ DNA into bacterial cells, anneal to each other to create a circular genome which can be replicated and which is not susceptible to degradation by bacterial exonucleases as a linear DNA would be.

In this embodiment, prior to cloning heterologous DNA into a λ vector, the 3' ends of the λ vector are blocked for example by adding a dideoxynucleotide together with an appropriate DNA polymerase, for example, klenow fragment, which effectively removes the reactive OH-groups from the 3' cos sites, leaving only an unreactive hydrogen, which is unable to be either extended or tagged. Thus after the cloning reaction has been carried out, the DNA molecules are subjected to the addition of a tag capable of being added only to 3' OH groups. In this way, it is only the correctly ligated products, which do not include a reactive 3' OH group which will not be tagged; all other DNA molecules, including the restricted vector fragments and the fragment to be cloned, will have a reactive 3' OH group which can be tagged. Since it is only the correctly ligated DNA molecules which cannot be tagged, these may easily be separated from the unwanted by products using any of the methods described herein.

Thus viewed from a further aspect, the present invention provides a method of in vitro phage packaging of recombinant phage wherein the vector DNA is cut with one or more restriction enzymes, 3' OH groups of the vector DNA are blocked, vector and DNA to be inserted are contacted under conditions appropriate for ligation of DNA fragments, and the ligation products are tagged with a moiety capable of attaching to reactive 3' OH groups, followed by separation of tagged and untagged molecules.

In this context, blocking of the 3'OH groups means that the 3'OH group is absent, or is protected or modified in some way such that it cannot be further extended.

In one convenient embodiment, the label is biotin, which is added to the reactive 3' OH groups in the form of a biotinylated nucleotide by enzymatic means, for example by means of klenow fragment.

In one embodiment, the 3' ends of the vector are first blocked, for example with a dideoxynucleotide, and the vector then cut with an enzyme that has a single recognition site within the vector, the recognition site being located between the two restriction sites to be used for cloning. An example of such a restriction enzyme is EcoRI. The purpose of this initial restriction enzyme cutting is to enable all vector DNA molecules which are not subsequently cut to be removed by tagging. The vector is then cut in two positions, with two enzymes that have unique cutting sites within the vector which will form the cloning site. In addition to generating the vector fragments for cloning, a number of partially cut restriction products may be obtained which can be removed by means of the tag, for example , where the label is biotin, by means of streptavidin binding, as described above. The DNA fragment(s) for cloning are then ligated into the lambda vector DNA. To remove all unwanted reaction products, all exposed 3' ends are labelled, for example by means of biotin. The correct ligation products do not have reactive 3' OH groups and cannot be tagged, and may thus be separated from all tagged molecules by the methods described herein.

The invention will now be described in more detail with reference to the following non-limiting Examples.

### EXAMPLES

### Example 1

### Endlabelling of restricted DNA fragments with a 5'-protruding DNA end

### Reagents:

5 µg lambda HindIII restricted DNA (Gibco 15612-13)
40 units DNA polymerase I, Large Klenow fragment (New England Biolabs #210S)
10 *µ*l buffer for DNA polymerase I, Large Klenow fragment (New England Biolabs)
5 nmol biotin-14-dCTP (Gibco 19518-018)
5 nmol dATP (Gibco 10216-018)
5 nmol dTTP (Gibco 10219-012)
5 nmol dGTP (Gibco 10218-014)
Distilled water ad 100 µl

The reaction mixture was incubated at 25°C for 45 minutes.

The reaction mixture was passed through a S400 HR MicroSpin column (Pharmacia-Amersham 275140-01), 10 µg streptavidin (Promega #7041) was added and incubated 5 minutes at 25°C.

The reaction mixture was added to a Centriflex column (Edge Biosystems #73883) and allowed to diffuse through the membrane as described by manufacturer, before centrifugation at 10000xg for 30 seconds. 10 µl distilled water was added to the membrane which was turned horizontally 180° and centrifuged again at 10000xg.

The eluate was analysed on 1% agarose (FMC #50080) gel electrophoresis, stained with ethidium bromide as described in Maniatis, Molecular Cloning: a laboratory manual 2nd ed 1989.

No trace of lambda DNA could be seen on the gel indicating that the end labelled linear molecules with streptavidin attached were retained on the membrane.

### Example 2

### Endlabelling of a blunt-end and 3'-protruding DNA end fragments

### Reagents:

2 µg Low DNA Mass™ Ladder (Gibco 10068-013)
40 units Terminal Deoxynucleotidyl Transferase, Recombinant (Gibco 10533-016)
20 µl buffer Terminal Deoxynucleotidyl Transferase, Recombinant (Gibco)
5 nmol biotin-14-dCTP (Gibco 19518-018)
Distilled water ad 100 µl

The reaction mixture was incubated at 37°C for 45 minutes.

The reaction mixture was passed through a S200 HR MicroSpin column (Pharmacia-Amersham 275120-01) and 10 µg streptavidin (Promega #7041) was added. The mixture was incubated 5 minutes at 25°C.

The reaction mixture was added to a Centriflex column (Edge Biosystems #73883) and allowed to diffuse through the membrane as described by manufacturer, before centrifugation at 10000xg for 30 seconds. 10 µl distilled water was added to the membrane which was turned horizontally 180° and centrifuged again at 10000xg.

For inspection, the eluate was run on 2% agarose (FMC #50080) gel electrophoresis, which was stained with ethidium bromide as described in Maniatis, Molecular Cloning: a laboratory manual 2nd ed. 1989.

No traces of DNA could be seen on the gel indicating that the end labelled linear molecules with streptavidin attached were retained on the membrane.

### Example 3

### Preparing vector DNA by removal of stuffer fragment

Cantab 5E vector containing a test insert (Pharmacia-Amersham 279401-01) was prepared using Qiagen maxiprep (Qiagen #12166)

Cantab 5E vector contains unique restriction sites for the enzymes SfiI, NotI and BsmI, with the SfiI and BsmI sites being located on either side of the NotI site

### Reagents:

25 µg Cantab 5E vector as described above
40 units of NotI endonuclease (Boehringer-Mannheint 1014714)
5 µl buffer for NotI endonuclease (Boehringer-Mannheim 1014714)
water ad 50 µl

The mixture was incubated 1 hour at 37°C.

The reaction mixture was passed through a S400 HR MicroSpin column (Pharmacia-Amersham 275140-01) whereafter the following were added:
40 units DNA polymerase I, Large Klenow fragment (New England Biolabs #210S)
10 *µ*l buffer for DNA polymerase I, Large Klenow fragment (New England Biolabs)
5 nmol biotin-14-dCTP (Gibco 19518-018)
5 nmol dGTP (Gibco 10218-014)
Distilled water ad 100 µl

The mixture was incubated at 25°C for 45 minutes.

The reaction mixture was passed through a S400 HR MicroSpin column (Pharmacia-Amersham 275140-01).

A sample of the reaction mixture (5 µg) was diluted to 50 µl and added to a Centriflex column (Edge Biosystems #73883) and allowed to diffuse through the membrane as described by manufacturer, before centrifugation at 10000xg for 30 seconds. 10 µl distilled water was added to the membrane which was turned horizontally 180° and centrifuged again at 10000xg.

For inspection, the eluate was run on 2% agarose (FMC #50080) gel electrophoresis, which was stained with ethidium bromide as described in Maniatis, Molecular Cloning: a laboratory manual 2nd ed. 1989.

No traces of Vector DNA were seen on the gel indicating that all the DNA was retained on the membrane.

The reaction mixture was passed through a S400 HR MicroSpin column (Pharmacia-Amersham 275140-01), and individual aliquots treated as follows:

### (1)

33 µl of the reaction mixture were placed in a separate tube (1) to which was added:
20 units BsmI (Boehringer-Mannheim 1292315)
5 µl BsmI buffer (Boehringer-Mannheim 1292315)
distilled water ad 50 µl

The mixture was incubated for 1 hour at 65°C.

### (2)

33 µl portion of the reaction mixture were placed in a separate tube (2) to which was added:
20 units SfiI (Boehringer-Mannheim 1288032)
5 µl SfiI buffer (Boehringer-Mannheim 1288032)
distilled water ad 50 µl

The mixture was incubated for 1 hour at 50°C.

### (3)

33 µl portion of the reaction mixture were placed in a separate tube (3) to which was added:
20 units SfiI (Boehringer-Mannheim 1288032)
5 µl SfiI buffer (Boehringer-Mannheim 1288032)
distilled water ad 50 µl

The mixture was incubated for 1 hour at 50°C. Then the reaction mix was passed through a S400 MicroSpin column and the following were added to reaction mix:
20 units BsmI (Boehringer-Mannheim 1292315)
5 µl BsmI (Boehringer-Mannheim 1292315) buffer
distilled water ad 50 µl

The mixture was incubated for 1 hour at 65°C.

Each of the three reaction mixtures were purified on separate S400 MicroSpin columns, and 10 µg streptavidin (Promega #7041) were added to each of the mixtures which were incubated for 5 minutes at 25°C.

Each of the reaction mixtures were added to a Centriflex column (Edge Biosystems #73883) and allowed to diffuse through the membrane as described by manufacturer, before centrifugation at 10000xg for 30 seconds. 10 µl distilled water was added to each membrane and the membranes were turned horizontally 180° and centrifuged again at 10000xg.

The three reaction mixtures were run on 1% agarose (FMC #50080) gel, and stained with ethidium bromide.

Only from the third reaction sample could purified vector DNA be seen on the gel, samples 1 and 2 gave no detectable traces of DNA. This indicates that it is only the correct restricted vector which passed through the membrane.

### Example 4

### Restriction of biotinylated PCR fragment

A PCR product was produced amplifying a scFV construct using high-quality biotinylated PCR primers.

The scFV construct is an 800 base pair fragment, which has unique sites for SfiI and NotI, at 40 and 760 bases respectively.

1 µg of PCR product was mixed with 2 µg of streptavidin (Promega #7041) and incubated at 25°C for 5 minutes.

The reaction mix was added to a Centriflex column (Edge Biosystems #73883) and allowed to diffuse through the membrane as described by manufacturer, before centrifugation at 10000xg for 30 seconds. 10 µl distilled water was added to the membrane which was turned horizontally 180° and centrifuged again at 10000xg.

For inspection, the reaction mix was run on 2% agarose (FMC #50080) gel, and stained with ethidium bromide.

No traces of the PCR product was detected, indicating that this had been retained on the membrane.

Separate aliquots were then treated as follows:

### (1)

3 µg of the PCR product was added in a separate tube (1) to
20 units NotI endonuclease (Boehringer-Mannheim 1014714)
5 µl NotI buffer and
distilled water ad 50 µl
and incubated for 1 hour at 37°C.

### (2)

Another 3 µg of the PCR product was added in a separate tube (2) to
20 units SfiI (Boehringer-Mannheim 1288032)
5 µl SfiI buffer (Boehringer-Mannheim 1288032)
distilled water ad 50 µl
and incubated 1 hour at 50°C.

### (3)

Another 3 µg of the PCR product was added in a separate tube (3) to
20 units SfiI (Boehringer-Mannheim 1288032)
5 µl SfiI buffer (Boehringer-Mannheim 1288032)
distilled water ad 50 µl
and incubated 1 hour at 50°C.

Then the reaction was passed through a S400 MicroSpin column and the following were added to the reaction mix:
20 units NotI endonuclease (Boehringer-Mannheim 1014714)
5 µl NotI endonuclease Boehringer-Mannheim 1014714) buffer
distilled water ad 50 µl
and incubated for 1 hour at 37°C.

Each of the three reaction mixtures were purified on separate S400 MicroSpin columns, and 10 µg streptavidin (Promega #7041) were subsequently added to each of the mixtures which were incubated for 5 minutes at 25°C.

Each of the reaction mixtures were added to a Centriflex column (Edge Biosystems #73883) and allowed to diffuse through the membrane as described by manufacturer, before centrifugation at 10000xg for 30 seconds. 10 µl distilled water was added to each membrane, and the membranes were turned horizontally 180° and centrifuged again at 10000xg.

For inspection, the three reaction mixtures were run on 1% agarose (FMC #50080) gel, and stained with ethidium bromide.

Only from the reaction mix in tube 3 could purified PCR product be seen on the gel, tubes 1 and 2 gave no detectable traces of DNA, indicating that end-containing fragments had been retained on the membrane, and the internal fragment had passed through the membrane.

### Example 5

### Restriction of non-biotinylated PCR fragment

A PCR product was produced amplifying a scFV construct using high-quality biotinylated PCR primers.

1 µg of PCR product was mixed with 2 µg of streptavidin (Promega #7041) and incubated at 25°C for 5 minutes, as per Example 4.

The reaction mix was added to a Centriflex column (Edge Biosystems #73883) and allowed to diffuse through the membrane as described by manufacturer, before centrifugation at 10000xg for 30 seconds. 10 µl distilled water was added to the membrane which was turned horizontally 180° and centrifuged again at 10000xg.

For inspection, the reaction mix was run on 2% agarose (FMC #50080) gel, and stained with ethidium bromide.

The PCR product was detected on the gel, indicating that it had not been retained on the membrane.

The following were added to 3 µg PCR product:
40 units Terminal Deoxynucleotidyl Transferase, Recombinant (Gibco 10533-016)
20 µl buffer Terminal Deoxynucleotidyl Transferase, Recombinant (Gibco)
5 nmol biotin-14-dCTP (Gibco 19518-018)
Distilled water ad 100 µl
and incubated at 37°C for 45 minutes.

The reaction mixture was passed through a S200 HR MicroSpin column (Pharmacia-Amersham 275120-01), 10 µg streptavidin (Promega #7041) was added and incubated 5 minutes at 25°C.

The reaction mixture was added to a Centriflex column (Edge Biosystems #73883) and allowed to diffuse through the membrane as described by manufacturer, before centrifugration at 10000xg for 30 seconds. 10 µl distilled water was added, the column turned horizontally 180° and centrifuged again at 10000xg.

For inspection: the reaction mixture was run on 2% agarose (FMC #50080) gel, and stained with ethidium bromide.

No PCR product could be detected on gel, indicating that it had been retained on the membrane.

Three separate aliquots were treated as follows:

### (1)

3 µg of the PCR product was added in a separate tube (1) to
20 units NotI endonuclease (Boehringer-Mannheim 1014714)
5 µl NotI buffer and
distilled water ad 50 µl
and incubated for 1 hour at 37°C.

### (2)

Another 3 µg of the PCR product was added in a separate tube (2) to
20 units SfiI (Boehringer-Mannheim 1288032)
5 µl SfiI buffer (Boehringer-Mannheim 1288032)
distilled water ad 50 µl
and incubated 1 hour at 50°C.

### (3)

Another 3 µg of the PCR product was added in a separate tube (3) to
20 units SfiI (Boehringer-Mannheim 1288032)
5 µl SfiI buffer (Boehringer-Mannheim 1288032)
distilled water ad 50 µl
and incubated 1 hour at 50°C.

Then the reaction was passed through a S400 MicroSpin column and the following were added to the reaction mix:
20 units NotI endonuclease (Boehringer-Mannheim 1014714)
5 µl NotI endonuclease (Boehringer-Mannheim 1014714) buffer
distilled water ad 50 µl
and incubated for 1 hour at 37°C.

Each of the three reaction mixtures were purified on separate S400 MicroSpin columns, and 10 µg streptavidin (Promega #7041) subsequently added and incubated for 5 minutes at 25°C.

Each of the three reaction mixtures were added to a Centriflex column (Edge Biosystems #73883) and allowed to diffuse through the membrane as described by manufacturer, before centrifugation at 10000xg for 30 seconds. 10 µl distilled water was added to each membrane and the membranes were turned horizontally 180° and centrifuged again at 10000xg.

For inspection, the three reaction mixtures were run on 1% agarose (FMC #50080) gel, and stained with ethidium bromide.

Only from the reaction mix group 3 could purified PCR product be seen on the gel, tubes 1 and 2 gave no detectable traces of DNA, indicating that end containing fragments had been retained on the membrane, and the internal fragment had passed through the membrane.

### Example 6

### Removal of linear DNA from a population of circular and linear DNA molecules

The circular DNA starting material is the cloning vector pUC19 which has a unique HindIII site in the polylinker cloning site.
5 µg pUC19 vector DNA (New England Biolabs #301-1S) was added to:
40 units of HindIII restriction enzyme (New England Biolabs #104S)
5 µl buffer for HindIII restriction enzyme (New England Biolabs)
distilled water ad 50 µl
Incubated 1 hour at 37°C.

The reaction was passed through a S400 HR MicroSpin column and the reaction mix added to:
5 µl pUC19 vector DNA (New England Biolabs #301-1S)
40 units DNA polymerase I, Large Klenow fragment (New England Biolabs #210S)
10 µl buffer for DNA polymerase I, Large Klenow fragment (New England Biolabs)
5 nmol biotin-14-dCTP (Gibco 19518-018)
5 nmol dATP (Gibco 10216-018)
5 nmol dTTP (Gibco 10219-012)
5 nmol dGTP (Gibco 10218-014)
Distilled water ad 100 µl

The reaction mixture was incubated at 25°C for 45 minutes.

The reaction mixture was passed through a S400 HR MicroSpin column (Pharmacia-Amersham 275140-01), 10 µg streptavidin (Promega #7041) was added and incubated 5 minutes at 25°C.

The reaction mixture was added to a Centriflex column (Edge Biosystems #73883) and allowed to diffuse through the membrane as described by manufacturer, before centrifugration at 10000xg for 30 seconds. 10 µl distilled water was added, the column turned horizontally 180° and centrifuged again at 10000xg.

For inspection, the eluate was analysed on 1% agarose (FMC #50080) gel electrophoresis, stained with ethidium bromide as described in Maniatis, Molecular Cloning: a laboratory manual 2nd ed 1989.

Only circular vector was detected on the agarose (FMC #50080) gel, indicating that it had passed through the membrane, the linear DNA having been retained.

### Example 7

### In vitro packaging of lambda phage, using precut lambda vector

The precut lambda vector Uni-ZAP® XR (Stratagene, cat. #236612) is used.

### Reagents:

10 µg precut lambda vector
dideoxyguanosine triphosphate (200 µM final concentration)
dideoxyadenosine triphosphate (200 µM final concentration)
Klenow fragment (10 units)
NEB EcoPol buffer
in a total volume of 30 µl

The reaction mixture is incubated at 24°C for 20 minutes.

The reaction mixture is passed through a S400 HR MicroSpin column to remove proteins and nucleotides and to the purified reaction mix is added:
200 ng of pre-prepared DNA insert
T4 DNA ligase
NEB T4 ligase buffer
in a volume of 40 µl

The reaction mixture is incubated at 16°C for 48 hours.

The reaction mixture is passed through a S400 HR MicroSpin column and to the purified reaction mix is added:
Biotinylated dCTP (5 nmol)
Biotinylated dATP (5 nmol)
dTTP (200 µM final concentration)
dGTP (200 µM final concentration)
Klenow fragment (10 units)
NEB buffer
in a volume of 50 µl

The reaction mixture is incubated at 24°C for 20 minutes.

The reaction mixture is passed through a S400 HR MicroSpin column and to the purified reaction mix is added Streptavidin and the reaction mixture added to a Centriflex column as in Example 2.

Further processing follows the instructions of the packaging insert of Stratagene. The ligation gives a higher number of viable bacteriophages than found in the standard method, as seen by plaque count on bacteria.

### Example 8

### In vitro packing of lambda phage using uncut lambda vector

The uncut lambda vector Uni-ZAP® XR (Stratagene) containing a test insert is used.

### Reagents:

5 µg uncut lambda vector
dideoxyguanosine triphosphate (200 µM final concentration)
dideoxyadenosine triphosphate (200 µM final concentration)
Klenow fragment (10 units)
NEB buffer
in a total volume of 20 µl

The reaction mixture is incubated at 24°C for 20 minutes. This blocks the 3' ends of the vector.

The reaction mixture is passed through a S400 HR MicroSpin column and to the purified reaction mix is added:
EcoRI restriction enzyme (20 units)
NEB EcoRI buffer
in a volume of 30 µl.

The reaction mixture is incubated at 37°C for 120 minutes. This digests the vector into approximately equal pieces.

The reaction is passed through a S400 HR MicroSpin column and to the purified reaction mix is added:
Biotinylated dATP (5 nmol)
dTTP (200 µM final concentration)
dCTP (200 µM final concentration)
dGTP (200 µM final concentration)
Klenow fragment (10 units)
NEB buffer
in a volume of 40 µl

The reaction mixture is incubated at 24°C for 30 minutes. In this reaction, biotin will label the EcoRI digested lambda

To the reaction mixture is added:
Xbal and Xhol restriction enzymes (20 units of each) in
NEB 2 buffer in a volume of 50 µl. This reaction creates the cloning site.

The reaction mixture is incubated at 37°C for 60 minutes.

The reaction is passed through a S400 HR MicroSpin column and to the purified reaction mix is added Streptavidin and the reaction mixture added to a Centriflex column as in example 2. In this step, Streptavidin binds to uncut vector.

The reaction is passed through a S400 HR MicroSpin column and to the purified reaction mix is added:
200 ng of pre-prepared DNA insert
T4 DNA ligase
NEB T4 ligase buffer
in a volume of 60 µl

The reaction mixture is incubated at 16°C for 48 hours.

The reaction mixture is passed through a S400 HR MicroSpin column and to the purified reaction mix is added:
Biotinylated dCTP (5 nmol)
Biotinylated dATP (5 nmol)
dTTP (200 µM final concentration)
dGTP (200 µM final concentration)
Klenow fragment (10 units)
NEB buffer
in a volume of 70 µl

The reaction mixture is incubated at 24°C for 20 minutes. In this reaction, all exposed 3' OH ends not previously blocked are labelled.

The reaction mixture is passed through a S400 HR MicroSpin column and to the purified reaction mix is added Streptavidin and the reaction mixture added to a Centriflex column as in example 2. This separates the tagged from the untagged molecules.

Further processing follows the instructions of the packaging insert of Stratagene. The ligation gives a higher number of viable bacteriophages than found in the standard method, as seen by plaque count on bacteria.

### Example 9

### Clean-up of reamplification PCR reaction

### a) Modification of Cantab5E vector (Pharmacia) to obtain Cantab5E^{BamHI(1)}

Cantab5E vector (1 µg) is cut by adding 10 units of BamHI and 10 µl NEB BamHI buffer in of total reaction volume of 100 µl and incubated for 1 hour at 37°C. The reaction mixture is separated on 1% agarose gel and the fragment of highest molecular weight is isolated by common gel extraction method. To 500 ng of the isolated large fragment is added 1 Weiss unit of T4 DNA ligase and 5 µl NEB ligase buffer in a total reaction volume of 50 µl and the mixture is incubated for 1 hour at 24°C. The ligation mixture is transformed into TGI cells using electroporation and the bacterial cells are incubated over night on ampicillin-containing agar (10.0 µg/ml). DNA from colony-forming bacteria is isolated and verified by size and restriction enzymes to contain only one BamHI restriction site. This DNA is named Cantab5E BamHI (1)

### b) Using the modified vector Cantab5E^{BamHI(1)} as a template in PCR

### Reagents:

10 µg Cantab5E^{BamHI(1)} vector
dNTP mix (supplied by Finnzyme; 200 µM final concentration)
10 µl expand high fidelity buffer (Boehringer-Mannheim AG)
30 pmol primer 1 that is partly overlapping with a unique afiIII restriction site 30 pmol primer 2 that is partly overlapping with a unique BamHI restriction site water ad 100 µl
The PCR reaction was heated to 96°C for 2 minutes before adding 1 µl expand high fidelity enzyme (Boehringer-Mannheim AG).

The reaction mixture is cycled on a thermo-cycler for 20 rounds, at following conditions:
- First 2 minutes at 96°C, then 20 rounds as follows
- 30 seconds at 55°C,
- 80 seconds at 72°C,
- 30 seconds at 96°C.

The PCR product is identified by ethidiumbromide staining after electrophoresis on agarose. Typically unwanted by-products are observed.

The thermo-cycled reaction mixture is passed through a S400 HR MicroSpin column.

The purified PCR product is cut using restriction enzymes AflIII and BamHI in a normal procedure, followed by adding streptavidin, and the reaction mixture added to a Centriflex column (as in example 2). Only correctly cut PCR product is seen upon Agarose electrophoresis, as unwanted impurities are retained by the protein-binding matrix.

### Example 10

### Use of biotin- containing DNA in diagnostic PCR of sickle cell anaemia

Normal human hemoglobin A1 has a DNA sequence for the first exon (coding part) of the haplotype A1-beta-globin gene beginning as shown:

### Normal DNA:

atg gtg cac ctg act cct gag gag aag tct gcc gtt act gcc
ctg tgg ggc aag gtg aac gtg gat gaa gtt ggt ggt gag gcc
ctg ggc agg ...

which translates to the

### Normal aminoacid sequence:

MVHLTPEEKSAVTALWGKVNVDEVGGEALG...

For the human disease sickle cell anaemia, the DNA sequence for the first exon of haplotype S beta-globin gene begins as shown:

### Sickle cell disease DNA:

atg gtg cac ctg act cct gtg gag aag tct gcc gtt act gcc
ctg tgg ggc aag gtg aac gtg gat gaa gtt ggt ggt gag gcc
ctg ggc agg ...

which translates to the

### Sickle cell disease aminoacid sequence:

MVHLTPVEKSAVTALWGKVNVDEVGGEALG...

Comparison of normal and sickle cell disease DNA reveals that a single base substitution is sufficient to transform the normal hemoglobin gene to an abnormal hemoglobin gene, known to causing the serious condition of sickle cell anaemia. The gag codon is mutated to a gtg codon causing a replacement of the acidic aminoacid glutamic acid with the nonpolar aminoacid valine. To detect the presence or absence of this mutation, genomic DNA is isolated, and, if necessary, amplified by PCR prior to diagnostic PCR in the conventional manner.

### Example 10(a)Isolation of patient DNA

A commercial kit (GFX Genomic Blood DNA Purification kit; Pharmacia-Amersham #27-9603-01) is used according to manufacturers recommendation to isolate genomic DNA from a patient blood sample.

If sufficient DNA is obtained, one proceeds directly to Example 10c. If not, a PCR amplification step of part of the hemoglobin beta chain gene is performed (Example 10b).

### Example 10(b) Amplifying step (optional, if needed for lack of material)

The isolated DNA of Example 10a is amplified to increase the amount of DNA for further processing, as shown below.

A mixture of
250 ng genomic DNA
10 µl high expand fidelity buffer
20 nmol dNTP
20 pmol of each amplification primer (Primer 1 and Primer 2; see below)
Water to 100 µl
3 units of Expand High Fidelity PCR enzyme
is used for PCR for 30 rounds at the following standard conditions:
hotstart at 96°C
annealing at 56° for 30 seconds
polymerization at 72°C for 1 minute
denaturation 96°C for 30 seconds

The primers used for amplification of part of the hemoglobin beta chain gene are based on the DNA sequence given by EMBEL search program when searching on the unique identifier EMBL-ID:HSBETGLOB'.

The hemoglobin beta chain gene (shown below as separated triplets) is preceded by an intron sequence (intron 1) and followed by another sequence (intron 2), both introns are indicated below in italics.

Part of the introns (underlined) are used for construction of primers (see below).

gcataaaagtcagggcagagccatctattgcttacatttgcttctgacacaactgt
gttcactagcaacctcaaacagacacc atg gtg cac ctg act cct gag
gag aag tct gcc gtt act gcc ctg tgg ggc aag gtg aac gtg
gat gaa gtt ggt ggt gag gcc ctg ggc agg
ggcaggttggtatcaaggttacaagacaggtttaaggagaccaatagaaactgggc
atgtggagacagagaagactcttgggtttctgataggcactgactctctctgccta
ttggtctattttcccacc...

The non-coding introns are flanking the coding portion of the gene; the second intron separating the coding sequence from the next part of the coding sequence further downstream.

To amplify the first coding part of the gene for later diagnostic PCR, two primers may be used, for example, annealing to introns 1 and 2, respectively. Annealing sequences for the primers are indicated as *underlined* *italics* above.

Primer 1: 5'-ctagcaacctcaaacagacacc-3'

Primer 2: 5' -gtaaccttgataccaacctgcc-3'

The primers are only used in the amplifying PCR to gain more DNA template for the diagnostic PCR procedure, and thus need not be biotinylated or modified in any way.

### Example 10c Diagnostic PCR

### Step 1 - construction of primers

For the diagnostic PCR since the nature of the mutation is known, a single base mutation in codon 6 of the exon, three primers are designed, corresponding to normal and abnormal (Sickle cell anaemia) DNA:

### Primer corresponding to Normal hemoglobin:

PrimerN: 5'-atg gtg cac ctg act cct ga-biotin-OH

### Primer corresponding to the Sickle cell disease hemoglobin:

PrimerS:   5'-atg gtg cac ctg act cct gt-biotin-OH

### Primer for the other end of the gene

Primer2:    5'-gtaaccttgataccaacctgcc-3'

(This may be the same primer as used in the amplification step of Example 10b; it is not tagged or modified).

### Step 2 - diagnostic test with primers of step 1 and the PCR product from Example 10b

Two PCR reactions are performed, using the same conditions as in example 10b.

### a) PCR using the PrimerN + Primer 2:

100 ng PCR product DNA from Example 10b
10 µl high expand fidelity buffer
20 nmol dNTP
20 pmol of each amplification primer (Primer N and Primer2)
3 units of Expand High Fidelity PCR enzyme
water to 100 µl

### b) PCR using primers + Primer2:

as for PCR reaction (a) but with amplification primer AB replacing primer N

The PCR products from PCR reactions a) and b) are purified by the use of S400HR MicroSpin columns as described in Example 1. The products are then examined for presence of biotin by means of a centriflex membrane as described in Example 1. The reaction mixture is divided into two portions. Excess streptavidin (5 µg) is added to 9/10 of the reaction mixtures and incubated at 25°C for 5 minutes as in Example 1 then added to a centriflex membrane as in Example 1. The collected eluate is analysed on an agarose gel, as in Example 1, for the presence of a DNA product. The remaining 1/10 of the reaction mixture which has not been contacted with streptavidin is run in parallel on the agarose gel as a control sample.

The results from examining the two PCR reactions a) and b) can have one of four possible outcomes illustrated below.

| Biotinylated PCR product or not | | Interpretation (diagnosis) |
|---|---|---|
| PCR # a (primerN+primer2) | PCR # b (primerS+primer2) | |
| Yes | Yes | Both normal and Sickle cell variants present ("harmless" heterozygous condition) |
| No | No | No normal gene present (unknown mutation in codon 6; may represent another disease?) |
| Yes | No | Only normal gene present (no mutation in codon 6; healthy subject) |
| No | Yes | Only mutated variant (Sickle cell)in codon 6 (possible lethal homozygous condition) |

The method may also be used for diagnosis of multiple base mutations, as illustrated below, again in the case of sickle cell anaemia.

### Example 11

### Diagnostic PCR of sickle cell anaemia (multiple base mutation)

A multibase mutation (bold underlined)in the first exon of beta-globin gene is shown in the DNA sequence as shown: 
atg gtg cac ctg act cct aac gag aag tct gcc gtt act gcc
ctg tgg ggc aag gtg aac gtg gat gaa gtt ggt ggt gag gcc
ctg ggc agg ...
 which translates to the aminoacid sequence:

MVHLTPNEKSAVTALWGKVNVDEVGGEALG...

Although three bases have changed, a similar method as in Example 10 can be used to detect the mutation

As in Example 10, a PrimerN is used to reveal whether the normal gene is present. Two primers are constructed to diagnose the potential presence of a mutation.

One primer which corresponds to mutated hemoglobin as follows:

Primer AB2a
5'-atggtgcacctgactcctaac-biotin-OH

This primer identifies exactly the mutation 'acc' in codon 6.

The second primer is

Primer AB2b
5'-atggtgcacctgactccta-biotin-OH

This primer may identify all mutations in codon 6 starting with an 'a' base.

PCR reactions are performed with the normal PrimerN, and with one or more primers corresponding to (i.e. complementary to) the mutated sequence using the conditions of Example 10.

This method may be used for any mutation in a known sequence of the normal gene and the hemoglobin Sickle cell mutation is given as one example. Where the common mutations seen in the population are known, a number of primers may be used to pinpoint exactly the mutation that is present.

### Examples 12-22

The Examples 1-11 are repeated, with a NycoCard bearing a protein binding membrane replacing the centriflex column for removal or isolation of biotin-labelled and steptavidin-complexed DNA molecules.

## Claims

1. A method for at least partially separating nucleic acid molecules in a sample into populations wherein a population is tagged with a protein capable of being immobilised on a matrix, said method comprising contacting the nucleic acid containing sample with a matrix which selectively binds proteins whereby the said protein interacts directly with the matrix, whereby the tagged molecules are captured by the matrix and are thereby separated from untagged molecules.

2. A method as claimed in claim 1 wherein the tag is a protein which can be incorporated into a nucleic acid molecule or a protein which has an affinity for a nucleic acid molecule.

3. A method as claimed in any one of claims 1 to 2 wherein the tag is an antigen.

4. A method as claimed in claim 1 or claim 2 wherein the protein is a nucleic acid binding protein.

5. A method as claimed in claim 1 wherein the proteins are linked to or attached to the nucleic acid molecules.

6. A method as claimed in claim 5 wherein the protein is linked via biotin to the nucleic acid, and the protein is streptavidin or avidin.

7. A method as claimed in any one of the preceding claims wherein the matrix is in the form of sheets, gels, filters, membranes, fibres, tubes, microtitre plates, columns, particles.

8. A method as claimed in claim 7 wherein the matrix is a porous material.

9. A method as claimed in claim 8 wherein the matrix is incorporated into a separation device selected from centrifuge vials, microtiter plates, cartridges and syringes.

10. A method as claimed in any one of claims 7 to 9 wherein an absorbent pad is located on said porous material, a liquid impermeable sheet is located on the face of said absorbent pad remote from said porous material, and a liquid impermeable sheet having one or more holes therein is located on the face of said porous material remote from said absorbent pad, whereby the test sample is applied to one of said holes and is caused to diffuse transversely through said porous material by absorption into said absorbent pad.

11. A method as claimed in any one of the preceding claims for the at least partial separation of a product of restriction enzyme digestion, or for purifying the products of the PCR reaction.

12. A method as claimed in any one of the preceding claims for at least partially separating a mixture of restriction enzyme digested fragments of DNA wherein the starting material is a linear DNA molecule which is tagged or capable of being tagged at or near one or both ends with a protein capable of being immobilised on a matrix, said method comprising subjecting the DNA molecule to restriction enzyme digestion followed by contacting the sample with a matrix which selectively binds proteins whereby the tagged molecules which originate from an end of the starting material are captured by the matrix and are thereby separated from untagged molecules.

13. A method as claimed in any one of the preceding claims for at least partially separating the correct and desired products of PCR amplification from PCR products which result from incorrect annealing of a PCR primer to template, wherein the template nucleic acid molecule comprises a unique restriction enzyme recognition site at or towards an end of the template, and a PCR primer which is tagged or capable of being tagged with a protein is complementary to a sequence on the template which extends partially into the unique restriction site, the method comprising amplifying the template by means of PCR, digesting the PCR products with the restriction enzyme specific for the said unique restriction enzyme recognition site, and contacting the resulting product with a matrix which selectively binds proteins whereby tagged nucleic acid molecules are captured by the matrix and thereby separated from untagged molecules.

14. A method as claimed in claim 13 wherein the tag is attached at the 5' end of the primer.

15. A method as claimed in any one of claims 1 to 10 for diagnostic PCR.

16. A method as claimed in claim 15 of diagnostic PCR wherein a test sample is separately subjected to PCR reactions, in which the mutation, if present, is in the sequence to which the 3' primer is complementary, a first PCR reaction using a 3' primer complementary to the normal target nucleic acid and a second PCR reaction using a 3' primer complementary to the mutant target, wherein the 3' nucleotide of the mutant primer corresponds to one of the nucleotides which is mutated in the mutant nucleic acid, each of said 3' primers bearing a tag or being capable of being tagged on the 3' nucleotide of the primer, wherein the presence or absence of the tag in the PCR reaction products is detected.

17. A method as claimed in claim 15 of diagnostic PCR of mutations in a nucleic acid molecule wherein the presence or absence of a mutation in a nucleic acid sample is detected, wherein a 3' primer specific for either the normal or mutant nucleic acid is used, wherein said primer is complementary to a region of the nucleic acid where there is a base difference between the normal and mutated DNA with the 3' terminus of the primer corresponding to the position in the sample where there is a difference between the normal and mutant, the primer being tagged or capable of being tagged, whereby the presence or absence of the tag in the PCR product is detected.

18. A method as claimed in claim 15 of diagnostic PCR in which a test sample is subjected to PCR with primers complementary to the normal target DNA wherein the primer for extending at the 3' end of the target anneals to a sequence the 3' nucleotide of which is mutated in the mutant, the 3' primer bearing a tag or being capable of being tagged at or on the 3' nucleotide, wherein the presence of the tag in the PCR reaction product as detected.

19. A method as claimed in any one of claims 1 to 10 for separating linear from circular nucleic acid molecules in a sample said method comprising introducing a tag to an end of the linear nucleic acid molecules, wherein said tag is a protein which is capable of being immobilised on a matrix by direct interaction with the matrix and contacting the sample with a matrix which selectively binds proteins, whereby said tagged linear nucleic acid molecules are immobilised on the matrix.

20. A method as claimed in any one of claims 1 to 10 for in vitro phage packaging of recombinant phage wherein the vector DNA is cut with one or more restriction enzymes, 3' OH groups of the vector DNA are blocked, vector and DNA to be inserted are contacted under conditions appropriate for ligation of DNA fragments, and the ligation products are tagged with a moiety capable of attaching to reactive 3' OH groups, followed by separation of tagged and untagged molecules.

## Patentansprüche

1. Verfahren um Nukleinsäuremoleküle in einer Probe mindestens teilweise in Populationen zu trennen, wobei eine Population mit einem Protein markiert ist, das geeignet ist an einer Matrix immobilisiert zu werden, wobei das Verfahren umfasst: in Kontakt bringen der nukleinsäurehaltigen Probe mit einer Matrix die selektiv Proteine bindet, wobei das Protein direkt mit der Matrix wechselwirkt, wodurch die markierten Moleküle von der Matrix gefangen werden und dadurch von nicht markierten Molekülen abgetrennnt werden.

2. Verfahren nach Anspruch 1, wobei die Markierung ein Protein ist, das in ein Nukleinsäuremolekül eingebaut werden kann oder ein Protein, welches eine Affinität zu einem Nukleinsäuremolekül aufweist.

3. Verfahren nach einem der Ansprüche 1 bis 2, wobei die Markierung ein Antigen ist.

4. Verfahren nach Anspruch 1 oder Anspruch 2, wobei das Protein ein Nukleinsäure bindendes Protein ist.

5. Verfahren nach Anspruch 1, wobei die Proteine mit den Nukleinsäuremolekülen verknüpft sind mit oder an die Nukleinsäuremoleküle gebunden sind.

6. Verfahren nach Anspruch 5, wobei das Protein über Biotin mit der Nukleinsäure verknüpft ist, und das Protein Streptavidin oder Avidin ist.

7. Verfahren nach einem der vorhergehenden Ansprüche, wobei die Matrix in Form von Folien, Gelen, Filtern, Membranen, Fasern, Röhren, Microtiterplatten, Säulen, Partikeln ist.

8. Verfahren nach Anspruch 7, wobei die Matrix ein poröses Material ist.

9. Verfahren nach Anspruch 8, wobei die Matrix in eine Trennvorrichtung, ausgewählt aus Zentrifugengläsern, Microtiterplatten, Kartuschen und Spritzen, eingebaut ist.

10. Verfahren nach einem der Ansprüche 7 bis 9, wobei sich auf dem porösen Material ein Absorbierkissen befindet, eine flüssigkeitsundurchlässige Folie sich auf der Vorderseite des Absorbierkissens, abgewandt von dem porösen Material, befindet und eine flüssigkeitsundurchlässige Folie die ein oder mehrere Löcher darin aufweist sich auf der Vorderseite des porösen Materials befindet, abgewandt von dem Absorbierkissen, wobei die Testprobe einem dieser Löcher zugeführt wird und Diffusion quer durch das poröse Material hervorgerufen wird durch Absorption in das Absorbierkissen.

11. Verfahren nach einem der vorhergehenden Ansprüche für die mindestens teilweise Abtrennung eines Produkts von Restriktionsenzymabbau, oder für die Reinigung der Produkte der PCR-Reaktion.

12. Verfahren nach einem der vorhergehenden Ansprüche um mindestens teilweise eine Mischung von Restriktionsenzymabbau-Fragmenten einer DNA zu trennen, wobei das Ausgangsmaterial ein lineares DNA-Molekül ist, welches an oder in der Nähe von einem oder beiden Enden mit einem Protein markiert ist oder dazu geeignet ist mit einem Protein markiert zu werden, das geeigent ist an einer Matrix immobilisiert zu werden, wobei das Verfahren unterziehen des DNA-Moleküls einem Restriktionsenzymabbau umfasst, gefolgt von in Kontakt bringen der Probe mit einer Matrix, welche selektiv Proteine bindet, wobei die markierten Moleküle die von einem Ende des Ausgangsmoleküls stammen von der Matrix gefangen werden und dadurch von nicht markierten Molekülen getrennnt werden.

13. Verfahren nach einem der vorhergehenden Ansprüche, um mindestens teilweise die richtigen und gewünschten Produkte der PCR-Amplifikation von PCR-Produkten die von inkorrekter Anlagerung eines PCR-Primers an Templat herrühren zu trennen,
wobei das Templat-Nukleinsäuremolekül umfasst: eine individuelle Restriktionsenzym-Erkennungsstelle an oder in der Nähe von einem Ende des Templats und einen PCR-Primer, der mit einem Protein markiert ist oder geeignet ist mit einem Protein markiert zu werden und komplementär ist zu einer Sequenz auf dem Templat das sich teilweise in die individuelle Spaltstelle erstreckt,
wobei das Verfahren umfasst: Amplifikation des Templats mittels PCR, Digerieren der PCR-Produkte mit dem Restriktionsenzym, das spezifisch für die individuelle Enzymerkennungsstelle ist, und in Kontakt bringen des resultierenden Produkts mit einer Matrix, welche selektiv Proteine bindet, wodurch markierte Nukleinsäuremoleküle von der Matrix gefangen werden und dadurch von nicht markierten Molekülen getrennnt werden.

14. Verfahren nach Anspruch 13, wobei die Markierung an das 5'-Ende des Primers gebunden ist.

15. Verfahren nach einem der Ansprüche 1 bis 10 für diagnostische PCR.

16. Verfahren nach Anspruch 15 zur diagnostischen PCR, wobei eine Testprobe getrennt PCR-Reaktionen unterzogen wird, in welcher die Mutation, falls vorhanden, in der Sequenz vorliegt, zu der der 3' Primer komplementär ist, eine erste PCR-Reaktion unter Verwendung eines 3'-Primers, der komplementär ist zu der normalen Zielnukleinsäure und eine zweite PCR-Reaktion unter Verwendung eines 3'-Primers der komplementär ist zu dem mutierten Ziel,
wobei das 3'-Nukleotid einem der Nukleotide entspricht die mutiert sind in der mutierten Nucleinsäure,
wobei jeder der 3'-Primer eine Markierung trägt oder geeignet ist an dem 3'-Nukleotid des Primers markiert zu werden,
wobei die Anwesenheit oder Abwesenheit der Markierung in den PCR-Reaktionsprodukten nachgewiesen wird.

17. Verfahren nach. Anspruch 15 für diagnostische PCR von Mutationen in einem Nukleinsäuremolekül, wobei die Anwesenheit oder Abwesenheit einer Mutation in einer Nukleinsäureprobe nachgewiesen wird,
wobei ein 3' Primer verwendet wird, der entweder für die normale oder mutierte Nukleinsäure spezifisch ist, wobei der Primer komplementär ist zu einem Bereich der Nukleinsäure in dem es einen Basenunterschied zwischen der normalen und mutierten DNA gibt, wobei das 3'-Ende des Primers der Position in der Probe entspricht wo es einen Unterschied gibt zwischen der normalen und mutieren, der Primer markiert ist oder geeignet ist markiert zu werden,
wobei die Anwesenheit oder Abwesenheit der Markierung in dem PCR-Produkt nachgewiesen wird.

18. Verfahren nach Anspruch 15 für diagnostische PCR in welchem eine Testprobe PCR unterworfen wird mit Primem die komplementär zu der normalen Ziel-DNA sind, wobei der Primer sich zur Verlängerung an dem 3'-Ende des Ziels an eine Sequenz anlagert deren 3' Nukleotid in dem Mutanten mutiert ist, der 3'-Primer eine Markierung trägt oder geeigent ist bei oder an dem 3'-Nukleotid markiert zu werden,
wobei die Anwesenheit der Markierung in dem PCR-Reaktionsprodukt nachgewiesen wird.

19. Verfahren nach einem der Ansprüche 1 bis 10 um lineare von zirkuläre Nukleinsäuremolekülen in einer Probe zu trennen, wobei das Verfahren umfasst: Einführen einer Markierung an einem Ende der linearen Nukleinsäuremoleküle, wobei die Markierung ein Protein ist, das geeigent ist an einer Matrix immobilisiert zu werden durch direkte Wechselwirkung mit der Matrix und in Kontakt bringen der Probe mit einer Matrix, die selektiv Proteine bindet,
wobei die markierten linearen Nukleinsäuremoleküle an der Matrix immobilisiert werden.

20. Verfahren nach einem der Ansprüche 1 bis 10 für *in vitro* Phagen-Umhüllung von rekombinanten Phagen, wobei die Vektor-DNA mit einem oder mehreren Restriktionsenzymen gespalten wird, 3' OH-Gruppen der Vektor-DNA blockiert werden, Vektor und DNA die insertiert werden sollen unter Bedingungen in Kontakt gebracht werden, die geeignet sind zur Ligation von DNA-Fragmenten, und die Ligationsprodukte markiert sind mit einer Gruppe, die geeigent ist an reaktive 3' OH-Gruppen zu binden, gefolgt von Trennung von markierten und unmarkierten Molekülen.

## Revendications

1. Procédé pour séparer au moins partiellement des molécules d'acides nucléiques dans un échantillon en populations, dans lequel une population est marquée avec une protéine capable d'être immobilisée sur une matrice, ledit procédé comprenant la mise en contact d'un échantillon contenant l'acide nucléique avec une matrice qui lie sélectivement les protéines, moyennant quoi ladite protéine interagit directement avec la matrice, moyennant quoi les molécules marquées sont capturées par la matrice et sont ainsi séparées des molécules non marquées.

2. Procédé selon la revendication 1, dans lequel le marquage est une protéine qui peut être incorporée à une molécule d'acide nucléique ou une protéine qui possède une affinité pour une molécule d'acide nucléique.

3. Procédé selon l'une quelconque des revendications 1 à 2, dans lequel le marquage est un antigène.

4. Procédé selon la revendication 1 ou la revendication 2, dans lequel la protéine est une protéine de liaison d'acide nucléique.

5. Procédé selon la revendication 1, dans lequel les protéines sont reliées ou fixées aux molécules d'acide nucléique.

6. Procédé selon la revendication 5, dans lequel la protéine est reliée via de la biotine à l'acide nucléique, et la protéine est de la streptavidine ou de l'avidine.

7. Procédé selon l'une quelconque des revendications précédentes, dans lequel la matrice est sous la forme de feuilles, de gels, de filtres, de membranes, de fibres, de tubes, de plaques de micro-titration, de colonnes, de particules.

8. Procédé selon la revendication 7, dans lequel la matrice est un matériau poreux.

9. Procédé selon la revendication 8, dans lequel la matrice est incorporée à un dispositif de séparation choisi parmi des flacons centrifuges, des plaques de micro-titration, des cartouches et des seringues.

10. Procédé selon l'une quelconque des revendications 7 à 9, dans lequel un tampon absorbant est situé sur ledit matériau poreux, une feuille imperméable au liquide est située sur la face dudit tampon absorbant à l'écart dudit matériau poreux, et une feuille imperméable au liquide possédant un ou plusieurs trous à l'intérieur est située sur la face dudit matériau poreux à l'écart dudit tampon absorbant, moyennant quoi l'échantillon de test est appliqué sur l'un desdits trous et est diffusé transversalement au travers dudit matériau poreux par absorption dans ledit tampon absorbant.

11. Procédé selon l'une quelconque des revendications précédentes pour la séparation au moins partielle d'un produit de digestion d'enzyme de restriction, ou pour purifier les produits de la réaction d'amplification en chaîne par polymérase (PCR).

12. Procédé selon l'une quelconque des revendications précédentes pour séparer au moins partiellement un mélange de fragments d'ADN digérés par enzyme de restriction, dans lequel la matière de départ est une molécule d'ADN linéaire qui est marquée ou capable d'être marquée au niveau de ou près d'une ou près des deux extrémités avec une protéine capable d'être immobilisée sur une matrice, ledit procédé comprenant la soumission de la molécule d'ADN à la digestion par une enzyme de restriction suivie de la mise en contact de l'échantillon avec une matrice qui lie sélectivement les protéines, moyennant quoi les molécules marquées qui proviennent d'une extrémité de la matière de départ sont capturées par la matrice et sont ainsi séparées des molécules non marquées.

13. Procédé selon l'une quelconque des revendications précédentes pour séparer au moins partiellement les produits corrects et souhaités de l'amplification PCR des produits PCR qui proviennent d'un annelage incorrect d'une amorce PCR avec une matrice, dans lequel la molécule d'acide nucléique de la matrice comprend un site de reconnaissance unique d'enzyme de restriction au niveau de ou vers une extrémité de la matrice, et dans lequel une amorce PCR qui est marquée ou qui est capable d'être marquée avec une protéine est complémentaire à une séquence sur la matrice qui s'étend partiellement dans le site de restriction unique, le procédé comprenant l'amplification de la matrice à l'aide d'une PCR, la digestion des produits de PCR avec l'enzyme de restriction spécifique audit site de reconnaissance unique d'enzyme de restriction, et la mise en contact du produit résultant avec une matrice qui lie sélectivement les protéines, moyennant quoi les molécules d'acide nucléique marquées sont capturées par la matrice et ainsi séparées des molécules non marquées.

14. Procédé selon la revendication 13, dans lequel le marquage est relié à l'extrémité 5' de l'amorce.

15. Procédé selon l'une quelconque des revendications 1 à 10 pour une PCR de diagnostic.

16. Procédé selon la revendication 15 de PCR de diagnostic, dans lequel un échantillon de test est séparément soumis à des réactions PCR, dans lesquelles la mutation, si elle est présente, se trouve dans la séquence à laquelle l'amorce 3' est complémentaire, une première réaction PCR utilisant une amorce 3' complémentaire à l'acide nucléique cible normal et une deuxième réaction PCR utilisant une amorce 3' complémentaire à la cible mutante, dans lequel le nucléotide 3' de l'amorce mutante correspond à l'un des nucléotides qui est muté dans l'acide nucléique mutant, chacune des dites amorces 3' portant un marquage ou étant capable d'être marquées sur le nucléotide 3' de l'amorce, dans lequel la présence ou l'absence du marquage dans les produits de réaction PCR est détectée.

17. Procédé selon la revendication 15 de PCR de diagnostic de mutations dans une molécule d'acide nucléique, dans lequel la présence ou l'absence d'une mutation dans un échantillon d'acide nucléique est détectée, dans lequel une amorce 3' spécifique à l'acide nucléique normal ou mutant est utilisée, dans lequel ladite amorce est complémentaire à une région de l'acide nucléique dans laquelle il existe une différence de base entre l'ADN normal et muté avec le 3' terminus de l'amorce correspondant à la position dans l'échantillon à laquelle il existe une différence entre le normal et le mutant, l'amorce étant marquée ou capable d'être marquée, moyennant quoi la présence ou l'absence du marquage dans le produit PCR est détectée.

18. Procédé selon la revendication 15 de PCR de diagnostic, dans lequel un échantillon de test est soumis à une PCR avec des amorces complémentaires à l'ADN cible normal, dans lequel l'amorce en vue de l'extension à l'extrémité 3' de la cible s'annelle à une séquence, dont le nucléotide 3' est muté dans le mutant, l'amorce 3' portant un marquage ou étant capable d'être marquée au niveau de ou sur le nucléotide 3', dans lequel la présence du marquage dans le produit de réaction PCR est détectée.

19. Procédé selon l'une quelconque des revendications 1 à 10 pour séparer des molécules d'acide nucléique linéaires de molécules d'acide nucléique circulaires dans un échantillon, ledit procédé comprenant l'introduction d'un marquage à une extrémité des molécules d'acide nucléique linéaires, dans lequel ledit marquage est une protéine qui est capable d'être immobilisée sur une matrice par interaction directe avec la matrice et en mettant en contact l'échantillon avec une matrice qui lie sélectivement les protéines, moyennant quoi lesdites molécules d'acide nucléique linéaires marquées sont immobilisées sur la matrice.

20. Procédé selon l'une quelconque des revendications 1 à 10 pour l'empaquetage phagique in vitro de phages recombinants, dans lequel l'ADN vecteur est découpé avec un ou plusieurs enzymes de restriction, les groupes OH 3' de l'ADN vecteur sont bloqués, le vecteur et l'ADN à insérer sont mis en contact dans des conditions appropriées pour la ligature des fragments d'ADN, et les produits de la ligature sont marqués avec un groupe capable de se lier aux groupes OH 3' réactifs, suivis de la séparation des molécules marquées et non marquées.
